(19) Europäisches Patentamt
European Patent Office
Office européen des brevets

(11) **EP 1 359 936 B1**

(12) **EUROPEAN PATENT SPECIFICATION**

(45) Date of publication and mention
of the grant of the patent:
**20.02.2008 Bulletin 2008/08**

(21) Application number: **02710762.2**

(22) Date of filing: **05.02.2002**

(51) Int Cl.:
***A61K 38/48*** *(2006.01)*     ***A61K 38/37*** *(2006.01)*

(86) International application number:
**PCT/DK2002/000081**

(87) International publication number:
**WO 2002/062377 (15.08.2002 Gazette 2002/33)**

(54) **COMBINED USE OF FACTOR VII POLYPEPTIDES AND FACTOR VIII POLYPEPTIDES**

KOMBINIERTE VERWENDUNG VON FAKTOR VII POLYPEPTIDEN UND FAKTOR VIII POLYPEPTIDEN

UTILISATION COMBINEE DE POLYPEPTIDES DE FACTEUR VII ET DE POLYPEPTIDES DE FACTEUR VIII

(84) Designated Contracting States:
**AT BE CH CY DE DK ES FI FR GB GR IE IT LI LU MC NL PT SE TR**

(30) Priority: **05.02.2001 DK 200100186**

(43) Date of publication of application:
**12.11.2003 Bulletin 2003/46**

(73) Proprietor: **Novo Nordisk Health Care AG
8700 Küsnacht (CH)**

(72) Inventors:
• **KNUDSEN, Jens, Bjerre
DK-3520 Farum (DK)**
• **HEDNER, Ulla
S-216 18 Malmø (SE)**
• **RØJKJÆR, Rasmus
DK-2820 Gentofte (DK)**

(74) Representative: **Nielsen, Lars Balzer et al
Novo Nordisk A/S
Corporate Patents
Novo Allée
2880 Bagsvaerd (DK)**

(56) References cited:
**WO-A-01/85199**

• **C. VAN 'T VEER, K.G. MANN: "Regulation of tissue factor initiated thrombin generation by the stoichiometric inhibitors tissue factor pathway inhibitor, antithrombin-III, and heparin cofactor-II" JOURNAL OF BIOLOGICAL CHEMISTRY, vol. 272, no. 7, 1997, pages 4367-4377, XP001128166**
• **R.T.DAVIS: "Treatment of acquired factor VIII deficiency with rituximab" BLOOD, vol. 96, no. 11 p2, 2000, XP001109373**

**Description**

FIELD OF INVENTION

The invention relates to a pharmaceutical composition comprising a preparation of a factor VII or factor VII-related polypeptide and a preparation of a factor VII or factor VIII-related polypeptide. The invention also relates to a kit-of-parts for treatment of bleeding episodes comprising a preparation of a factor VI or factor VII-related polypeptide and a preparation of a factor VIII or factor VIII-related polypeptide. The invention also relates to use of a preparation of a factor VII or factor VII-related polypeptide and a preparation of a factor VIII or factor VIII-related polypeptide for the preparation of a medicament. Furthermore, the invention relates to methods for treating bleedings, reducing clotting time, enhancing haemostasis, reducing the number of administrations of coagulation factor protein needed to accomplish haemostasis, reducing the amount of administered coagulation factor protein needed to accomplish haemostasis, prolonging clot lysis time, increasing clot strength, and enhancing fibrin clot formation.

BACKGROUND OF INVENTION

Blood coagulation factor VII (FVII) is a plasma coagulation factor. Activated factor VII (FVIIa) initiates the normal haemostatic process by forming a complex with tissue factor (TF), exposed as a result of the injury to the vessel wall, which subsequently activates factors IX and X (FIX and FX) into their activated forms, factors IXa and Xa (FIXa and FXa). Factor Xa converts limited amounts of prothrombin to thrombin on the tissue factor-bearing cell. Thrombin activates platelets and factors V and VIII into factors Va and VIIIa (FVa and FVIIIa), both cofactors in the further process leading to the full thrombin burst. This process includes generation of factor Xa by factor IXa (in complex with factor VIIIa) and occurs on the surface of activated platelets. Thrombin finally converts fibrinogen to fibrin resulting in formation of a fibrin clot.

Factor VII exists in plasma mainly as a single-chain zymogen, which is cleaved by FXa into its two-chain, activated form, FVIIa. Recombinant activated factor VII (rFVIIa) has been developed as a pro-haemostatic agent. The administration of rFVIIa offers a rapid and highly effective pro-haemostatic response in haemophilic subjects with bleedings who cannot be treated with coagulation factor products due to antibody formation. Also bleeding subjects with factor VII deficiency or subjects having a normal coagulation system but experiencing excessive bleeding can be treated successfully with FVIIa. In these studies, no unfavourable side effects of rFVIIa (in particular the occurrence of thromboembolism) has been encountered.

Blood coagulation factor VIII is a glycoprotein (MW 330,000) that circulates in blood. It is secreted by the liver and the endothelium and secreted into plasma where it circulates as a complex with von Willebrand factor. Factor VIII functions as a cofactor in blood coagulation in that it accelerates the conversion of factor X to factor Xa in the presence of factor IXa, calcium and phospholipid. Even though it is synthesized as a single polypeptide chain, it circulates in plasma primarily as a two-chain molecule. Activation of FVIII into an active cofactor requires additional proteolysis by thrombin or some other protease. A decrease in the presence or activity of factor VIII in the blood stream leads to haemophilia A. The level of the decrease in factor VIII activity is directly proportional to the severity of the disease. The current treatment of haemophilia A consists of the replacement of the missing protein by plasma-derived or recombinant factor VIII (so-called FVIII substitution or replacement treatment or therapy).

Coagulation factor deficiencies (e.g., FVIII deficiency) reflect different types of gene defects. Where the genetic lesion is severe, such as, deletion or frame shift, mRNA is not produced and (severe) deficiency results. Less severe genetic lesions from, for instance, point mutations which are not critically located result in secretion of protein with reduced biological activity. The inheritance pattern is recessive and X-linked, meaning that only men having one X-chromosome are affected. The severity of the coagulation defect can be mild or severe. Severity depends on the concentration of normally functioning factor VIII in plasma. The aim of replacement therapy is to raise the level of the patient's clotting factor activity (hereinafter called the "factor level") to one that will bring around haemostasis and to maintain it until healing is substantially complete. If the initiation of effective treatment is delayed, wound healing may be impaired and more treatment than usual will be required. The amount of treatment depends upon the plasma concentration of the coagulation factor needed for haemostasis, the recovery in blood and the half-life of the transfused material.

The level of factor VIII may also be more or less reduced in some subjects (e.g., women being carriers of the disease) who are heterozygous for the gene defect. Such subjects may have an increased bleeding tendency comparable to that of mildly-affected haemophilia patients and may be treated accordingly.

Some patients receiving factor VIII replacement therapy (having haemophilia A) develop an tibodies against the administered factor VIII. However, persons bom with a normal factor VIII level (not having a congenital factor VIII-deficiency) may for unknown reasons later in life develop auto-antibodies against factor VIII (acquired haemophilia A). In both cases the antibodies may be present in low, medium or high titres. In case of patients having a low or medium inhibitor-titre, these may sometimes be treated with factor VIII.

Haemophilia occurs in all degrees of severity. The patient with no detectable or less than 1 % factor VIII is usually severely affected and bleeds into muscles and joints on minimal trauma and sometimes apparently spontaneously. A small amount of factor VIII gives considerable protection so that patients with 1-5% of normal level factor VIII usually suffer only posttraumatic bleeding and less severe bleeding into muscles and joints, etc., and are often said to be moderately affected. Patients with more than 5% of factor VIII usually bleed only after significant trauma or surgery and are said to be mildly affected. It must be realised that this classification is not always valid in individual cases. Some patients with very low factor VIII levels rarely bleed whilst others even with over 5% factor VIII may bleed repeatedly into the "target joint" damaged originally by a traumatic haemarthrosis and appear to be "severely" affected. As a generalisation, however, bleeding symptoms are less obvious with higher factor levels so that abnormal bleeding does not usually occur at factor VIII levels over 35-40% of normal level. The general correlation between factor levels and symptoms in haemophilia A is shown below.

Severity of haemophilia related to factor VIII levels:

| Severity | Factor Level (% of normal level) | Type of presentation |
| --- | --- | --- |
| Severe | 0-1 | Apparently spontaneous bleeds. Severe bleeding |
| Moderate | 1-5 | Few bleeds. Haemarthroses mainly traumatic |
| Mild | 5-30 | Post-traumatic, post-surgical, post-dental extraction bleeding. Few episodes. |

The current treatment of haemophilia A consists of the replacement of the missing protein by plasma-derived or recombinant factor VIII. Factor VIII products are used as I.V. infusion (or injection) to treat acute bleeds on demand. The bleeding types are categorised as follows:

1. Haemarthrosis (bleeding in joints)
2. Life-and limb threatening bleeds (retroperitoneal bleeds, CNS bleeds, retropharyngeal bleeds, muscular bleeds with compartment syndrome and massive GI bleeds)
3. Bleeding prevention in relation to surgery (orthopaedic, elective procedures, emergency surgery)

Experience has shown that if factor VIII levels are maintained over 30-40% of normal level until healing is complete then normal haemostasis is usually maintained. However other considerations are also important. Movement of the affected parts such as a haemarthrosis, coughing or walking after abdominal surgery may promote bleeding. Physiotherapy or manipulation may require rather high levels whilst immobilisation of mild lesions may allow control of bleeding with relatively low factor levels. Approximate target levels which can be aimed for in various situations are shown below:

*Treatment of standard haemarthrosis (category 1):*
The normal intent is to achieve an initial factor VIII plasma concentration of at least 30 - 40 % of normal level followed by a plasma concentration of at least 10 - 20 % of normal level for 2 - 3 days.
*Treatment of Life-and limb threatening bleeds (category 2):*
The normal intent is to achieve an initial factor VIII plasma concentration of at least 50 % followed by a plasma concentration of at least 20 % for one week.
*Bleeding prevention in relation to surgery (category 3):*
The normal intent is to achieve a factor VIII plasma concentration of at least 60 - 100 % on the day of surgery followed by a plasma concentration of at least 30 - 40 % from day 2 to 7 and continuing with a plasma concentration of at least 10 - 20 % for one to two weeks.

Following the above guidelines for treatment, the following can be said of the number of factor VIII injections in relation to types of bleedings.
With an average plasma half-life of factor VIII of 10-12 hours the following average numbers of injections of factor VIII per bleeding episode are normally used in clinical praxis:

*Haemarthrosis (bleeding in joints):* Home treatment, minor haemarthrosis: 1-3 injections; Hospital treatment, larger haemarthrosis: 6 -14 injections.
*Life-and limb threatening bleeds:* 10 - 20 injections.
*Bleeding prevention in relation to surgery:* 30 - 40 injections

In clinical treatment of haemophilia FVIIa is presently used to stop bleedings in patients having inhibitors to FVIII or FIX (which prevents replacement therapy). However, clinicians do not normally use FVIIa as first line treatment for haemophiliacs without inhibitors (where FVIII or FIX, respectively, can be used) because it is expected that the short half-life of factor VIIa compared to that of factor VIII (2.5 hours compared to 10-12 hours) would require more frequent factor VIIa injections to maintaining a certain level of haemostatic ability.

European Patent No. 225.160 (Novo Nordisk) concerns compositions of FVIIa and methods for the treatment of bleeding disorders not caused by clotting factor defects or clotting factor inhibitors.

European Patent No. 82.182 (Baxter Travenol Lab.) concerns a composition of factor VIIa for use in counteracting deficiencies of blood clotting factors or the effects of inhibitors to blood clotting factors in a subject.

Lusher et al., Haemophilia, 1998, 4, pp.790-798 concerns the administration of recombinant factor VIIa in treatment of joint, muscle and mucotaneous haemorrhages in persons with haemophilia A and B, with and without inhibitors.

Kjalke et al, Thrombosis and Haemostasis, 1999 (Suppl), 095 1 concerns the administration of extra exogenous FVIIa and the effect on the formation of thrombin on the activated platelet surface in a model system mimicking haemophilia A or B conditions.

U.S. Patent No. 5,891,843 (Immuno) concerns a composition of FVIIa in combination with a second ingredient having FEIB-activity, e.g., activated prothrombin complex or a FEIBA preparation.

Today, many factor VIII products used in treatment of haemophilia contain recombinantly produced factor VIII. However, the products may also have been isolated from human or porcine plasma. These purified products often contain lesser amounts of other coagulation factors or other components from plasma. Normally, such additional plasma components are unwanted (due to risk of viral infection or other contamination), and the replacement of part of such products with a recombinant protein (e.g., factor VIIa) will be considered an improvement of the composition and treatment and a benefit to the patient.

Today, subjects having a reduced level of factor VIII (e.g., haemophilia A patients) experiencing bleeding episodes are generally treated with several injections, or infusions, of factor VIII before the bleeding is stopped. Furthermore, a considerable number of injections are needed to maintain haemostasis until the injury causing the bleeding is completely healed.

Trauma victims, suffering from excessive bleedings, are generally treated with large infusion volumes of fluids, such as fluids for intra venous (i.v.), injection colloid infusion products, albumin, red blood cell concentrates, etc. Extensive bleedings requiring massive blood transfusions may lead to the development of multiple organ failure including impaired lung and kidney function.

A faster arrest of bleedings would be an important benefit to such subjects. So would a reduction in the number of injections needed to stop bleeding and maintain haemostasis and or a reduction in the amount of coagulation protein usage for bleeding arrest and maintaining haemostasis.

There is still a need in the art for improved treatment of subjects experiencing bleeding episodes, including subjects where the bleeding episodes are due to a reduced level of coagulation factor VIII. There remains a need in the art for improved, reliable and widely applicable methods of enhancing coagulation, enhancing or ensuring formation of stable haemostatic plugs, enhancing convenience for the treated subject, or achieving full or sufficient haemostasis in subjects, in particular in subjects having an impaired thrombin generation. There is also a need for methods wherein the amount of FVIIa or the amount of FVIII needed for achieving full or sufficient haemostasis is lowered. There is also a need for methods wherein the total amount of coagulation factor protein needed for achieving full or sufficient haemostasis is lowered and methods wherein the time to bleeding arrest is shortened.

SUMMARY OF THE INVENTION

In a first aspect the invention concerns a pharmaceutical composition comprising (a) a preparation of human factor VIIa or an amino acid variant of human factor VIIa having no more than 20 amino acids replaced, deleted or inserted compared to wild-type factor VIIa; and (b) a preparation of human factor VIII or a truncated form of human factor VIII; wherein (a) and (b) are the sole haemostatic agents of the composition.

In a second aspect the invention concerns a kit-of-parts comprising

   a) an effective amount of a preparation of human factor VIIa or an amino acid variant of human factor VIIa having no more than 20 amino acids replaced, deleted or inserted compared to wild-type factor VIIa; and a pharmaceutically acceptable carrier in a first unit dosage form;
   b) an effective amount of a preparation of human factor VIII or a truncated form of human factor VIII, and a pharmaceutically acceptable carrier in a second unit dosage form; and
   c) container means for containing said first and second dosage forms;

wherein (a) and (b) are the sole haemostatic agents of the kit.

In another aspect the invention concerns the use of a preparation of (a) human factor VIIa or an amino acid variant of human factor VIIa having no more than 20 amino acids replaced, deleted or inserted compared to wild-type factor VIIa; in combination with a preparation of (b) human factor VII or a truncated form of human factor VIII; as the sole haemostatic agents for the manufacture of a medicament for treating bleeding episodes in a subject.

In one embodiment the ratio between the activity of the factor VIIa variant and the activity of native human factor VIIa (wild-type FVIIa) is at least about 1.25 when tested in the "In Vitro Hydrolysis Assay" as described in the present description.

In a further embodiment the human factor VIIa is recombinant human factor VIIa and in still further embodiment the human factor VIIa is plasma-derived factor VII.

In another embodiment the human factor VIII is recombinant factor VIII and in a further embodiment the human factor VIII polypeptide is plasma-derived factor VIII. In one series of embodiments of the invention the human factor VIII is in its activated form. In one embodiment the factor VIII is a fragment of factor VIII.

In one embodiment the human factor VIIa polypeptide and the human factor VIII polypeptide are present in a ratio by mass of between about 100:1 and about 1:100 factor VII:factor VIII.

The variants of human factor VIIa will be sequence variants having no more than 20 amino acids replaced, deleted or inserted compared to wild-type factor VIIa (i.e., a polypeptide having the amino acid sequence disclosed in U.S. Patent No. 4,784,950), In another embodiment, the factor VIIa variants have no more than 15 amino acids replaced, deleted or inserted; in another embodiment, the factor VIIa variants have no more than 10 amino acids replaced, deleted or inserted; in another embodiment, the factor VIaI variants have no more than 8 amino acids replaced, deleted or inserted; in another embodiment, the factor VIIa variants have no more than 6 amino acids replaced, deleted or inserted; in another embodiment, the factor VIIa variants have no more than 5 amino acids replaced, deleted or inserted; in another embodiment, the factor VIIa variants have no more than 3 amino acids replaced, deleted or inserted compared to wild-type factor VIIa. In one embodiment, the factor VIIa variants are selected from the list of L305V-FVIIa, L305V/M306D/D309S-FVIIa, L3051-FVIIa, L305T-FVIIa, F374P-FVIIa, V158T/M298Q-FVIIa, V158D/E296V/M298Q-FVIIa, K337A-FVIIa, M298Q-FVIIa, V158D/M298Q-FVIIa, L305V/K337A-FVIIa, V158D/E296V/M298Q/L305V-FVIIa, V158D/E296V/M298Q/K337A-FVIIa, V158D/E296V/M298Q/L305V/K337A-FVIIa, K157A-FVII, E296V-FVII, E296V/M298Q-FVII, V158D/E296V-FVII, V158D/M298K-FVII, and S336G-FVII.

The factor VIIa variants have increased tissue factor-independent activity compared to native human coagulation factor VIIa. The increased activity is not accompanied by changes in the substrate specificity. The binding of the factor VIIa variants to tissue factor should not be impaired and the factor VIIa variants should have at least the activity of wild-type factor VIIa when bound to tissue factor.

The clotting time is reduced in mammalian blood. In one embodiment the haemostasis is enhanced in mammalian blood. In another embodiment the clot lysis time is prolonged in mammalian blood. In another embodiment the clot strength is increased in mammalian blood. In another embodiment the fibrin clot formation is enhanced in mammalian blood. In one embodiment, the mammalian blood is human blood. In another embodiment, the mammalian blood is normal blood; in another embodiment, the mammalian blood is blood having a normal level of coagulation factor proteins; in another embodiment, the mammalian blood is blood having a normal level of factor VIII; in another embodiment, the blood is normal human blood; in one embodiment, the blood is blood from a subject having an impaired thrombin generation. In one embodiment, the blood is blood from a subject having a deficiency of one or more coagulation factors; in another embodiment, the blood is blood from a subject having inhibitors against one or more coagulation factors. In one embodiment, the blood is from a subject having a lowered concentration of fibrinogen. In one embodiment, the blood is factor VIII-deficient human blood.

The human factor VIa and the human factor VIII polypeptides are the sole haemostatic agents employed. "Sole" agents or factors as used herein refers to situations in which the factor VII or factor VII-related polypeptide and the factor VIII or factor VIII-related polypeptide, taken together, are the only haemostatic agents, active haemostatic agents, or coagulation factors, as applicable, contained in the pharmaceutical composition or kit, or are the only haemostatic agents, active haemostatic agents, or coagulation factors, as applicable, administered to the patient in the course of a particular treatment, such as, e.g., in the course of a particular bleeding episode. It will be understood that these situations encompass those in which other haemostatic agents or coagulation factors, as applicable, are not present in either sufficient quantity or activity so as to significantly influence one or more coagulation parameters.

In another embodiment, the pharmaceutical composition is formulated for intravenous administration. In one embodiment, the composition further contains a pharmaceutical acceptable excipient.

In one embodiment of the invention, the composition is in single-dosage form wherein the single-dosage form contains both coagulation factors. In one embodiment of the invention, the composition is in the form of a kit-of-parts comprising (a) a preparation of human factor VIIa or an amino acid variant of human factor VIIa having no more than 20 amino acids replaced deleted or inserted compared to wild-type factor VIIa; and

(b) a preparation of human factor VIII or a truncated form of human factor VIII.

In one embodiment of the invention, the human factor VIIa or an amino acid variant of human factor VIIa having no more than 20 amino acids replaced, deleted or inserted compared to wild-type factor VIIa; and human factor VIII or a truncated form of human factor VIII are administered in single-dosage form. In another embodiment of the invention, the human factor VIIa or an amino acid variant of human factor VIIa having no more than 20 amino acids replaced, deleted or inserted compared to wild-type factor VIIa and human factor VIII or a truncated form of human factor VIII are administered in the form of a first unit dosage form comprising the human factor VIIa or an amino acid variant of human factor VIIa having no more than 20 amino acids replaced, deleted or inserted compared to wild-type factor VIIa and a second unit dosage form comprising the human factor VIII or s truncated form of human factor VIII.

In one embodiment of the invention, the human factor VIIa polypeptide and the human factor VIII polypeptide are administered simultaneously. In another embodiment, the human factor VIIa polypeptide and the human factor VIII polypeptide are administered sequentially. In one embodiment, the human factor VIIa polypeptide and the human factor VIII polypeptide are administered with a time separation of no more than 15 minutes, preferably 10, more preferred 5, more preferred 2 minutes. In one embodiment, the human factor VIIa polypeptide and the human factor VIII polypeptide are administered with a time separation of up to 2 hours, preferably from 1 to 2 hours, more preferred up to 1 hour, more preferred from 30 minutes to 1 hour, more preferred up to 30 minutes, more preferred from 15 to 30 minutes.

In one embodiment, the effective amount of the human factor VIIa polypeptide is an amount from about 0.05 mg/day to about 500 mg/day (70-kg subject). In one embodiment, the effective amount of a preparation of a human factor VIII polypeptide is from about 0.01 mg/day to about 500 mg/day (70-kg subject).

In one embodiment the human factor VIIa polypeptide and the human factor VIII polypeptide are present in a ratio by mass of between about 100:1 and about 1:100 factor VII:factor VIII

In one embodiment of the present invention, the pharmaceutical composition is in single-dosage form and consists essentially of a preparation of the human factor VIIa or an amino acid variant of human factor VIIa having no more than 20 amino acids replaced, deleted or inserted compared to wild-type factor VIIa; and human factor VIII or a truncated form of human factor VIII and one or more of the components selected from the list of pharmaceutical acceptable excipients or carriers, stabilizers, detergents, neutral salts, antioxidants, preservatives, and protease inhibitors.

In a further embodiment, the subject is a human; in another embodiment, the subject has an impaired thrombin generation; in one embodiment, the subject has a lowered plasma concentration of fibrinogen (e.g., a multi-transfused subject); in one embodiment, the subject has a lowered plasma concentration of factor VIII.

In one embodiment, the pharmaceutical composition is for home treatment

In another aspect, the invention concerns the use of a preparation of human factor VIIa or an amino acid variant of human factor VIIa having no more than 20 amino acids replaced, deleted or inserted compared to wild-type factor VIIa; and human factor VIII or a truncated form of human factor VIII for the preparation of a medicament for the treatment of bleedings in a subject suffering from a factor VIII syndrome.

In another aspect, the invention concerns the use of a preparation of human factor VIIa or an amino acid variant of human factor VIIa having no more than 20 amino acids replaced, deleted or inserted compared to wild-type factor VIIa; and human factor VIII or a truncated form of human factor VIII for the preparation of a medicament for the treatment of bleedings in a subject having a reduced level of factor VIII.

In one embodiment, the subject to be treated has a reduced level of factor VIII. In one embodiment the subject suffers from a factor VIII-responsive syndrome. In one embodiment the factor VIII responsive syndrome is haemophilia A.

In one embodiment, the factor VIIa is human recombinant factor VIIa (rFVIIa). In another embodiment, the rFVIIa is NovoSeven® (Novo Nordisk A/S, Bagsvaerd, Denmark).

In one embodiment, the factor VIII is human recombinant factor VIII (rFVIII). In another embodiment, the factor VIII product is ReFacto (AHP/Genetics Institute), Alphanate (Alpha), Bioclate (Aventis), Monoclate-P (Aventis), Helixate (Aventis), Recombinate (Baxter), Hemofil M (Baxter), Kogenate (Bayer), Nordiate (HemaSure), FACTEUR VIII-LFB (Laboratoire Francais du Fractionnement et des Biotechnologies (LFB)), Hyate:C (Speywood), and Kogenate SF (Bayer).

In one embodiment, the pharmaceutical composition is formulated for intravenous administration. In one embodiment, the composition further comprises an inhibitor of the fibrinolytic system, including, without limitation, aprotinin, ε-aminocaproic acid or tranexamic acid.

LIST OF FIGURES

Figure 1: The clot shortening effect of rFVIIa in the absence and presence of FVIII in FVIII-deficient plasma is shown in figure 1.
Figure 2: The clot shortening effect of rFVIIa in the absence and presence of FVIII in normal human plasma is shown in figure 2.

DETAILED DESCRIPTION OF INVENTION

Subjects, who bleed excessively in association with surgery or major trauma thus needing blood transfusions, develop more complications than those who do not experience any bleeding. However, also moderate bleedings may lead to complications if they require the administration of human blood or blood products (platelets, leukocytes, plasma-derived concentrates for the treatment of coagulation defects, etc.) because this is associated with the risk of transferring human viruses (e.g., hepatitis, HIV, parvovirus, or other, by now unknown viruses) as well as non-viral pathogens. Extensive bleedings requiring massive blood transfusions may lead to the development of multiple organ failure including impaired lung and kidney function. Once a subject has developed these serious complications a cascade of events involving a number of cytokines and inflammatory reactions is started making any treatment extremely difficult and unfortunately often unsuccessful. Therefore, a major goal in surgery as well as in the treatment of major tissue damage is to avoid or minimise the bleeding. To avoid or minimize such unwanted bleedings it is important to ensure formation of stable and solid haemostatic plugs that are not readily dissolved by fibrinolytic enzymes. Furthermore, it is of importance to ensure quick and effective formation of such plugs or clots.

Subjects with thrombocytopenia (lowered count or activity of platelets) also have an impaired thrombin generation as well as a defective stabilization of the fibrin plugs resulting in haemostatic plugs prone to premature dissolution. Furthermore, subjects subjected to major trauma or organ damage and who, as a consequence, have obtained frequent blood transfusions often have lowered platelet counts as well as lowered levels of fibrinogen, factor VIII, and other coagulation proteins. These subjects experience an impaired (or lowered) thrombin generation. These subjects, therefore, have a defective, or less efficient, haemostasis leading to the formation of fibrin plugs that are easily and prematurely dissolved by proteolytic enzymes, such enzymes in addition being extensively released in situations characterized by extensive trauma and organ damage.

A patient experiencing a major loss of blood becomes clinically unstable. Such patient are in risk of experiencing atrial fibrillation, which may lead to a fatal stop of cardiac activity; impaired renal function; or fluid extravasations in lungs (so-called "wet lungs" or ARDS).

Bleedings in tissues may also lead to the formation of haematomas. The sizes of (in particular intercranial and spinal) haematomas are closely correlated to the extent of loss of neurological function, rehabilitation difficulties, and/or the severity and degree of permanent impairments of neurological function following rehabilitation. The most severe consequences of haematomas are seen when they are located in the brain where they may even lead to the death of the patient. The so-called compartment syndrome is a clinical condition caused by heavy bleeding internally into an extremity. In arms and legs the muscles and bones are externally confined by an almost inelastic collagen sheet called the fascia. Bleeding in spaced confined by the facia will lead to increased pressure in that compartment and subsequent pressure to nerves, vessels and muscle tissues, thus causing extensive tissue necrosis if not treated immediately. If formed, necrotic tissue will to a large extent, during the event of healing, be transformed into connective tissue, which is contracted compared to the original muscle tissue. Such contractures make the subject liable to experience impaired motility of affected joints which again leads to the need of corrective surgery. Severe haematomas may furthermore lead to formation of pseudo cysts which may be likened to benign tumours in that such cysts, like tumours, erode the affected muscle or bone tissues. Again, surgery is needed to remove such pseudo cysts.

Formation of haematomas furthermore increases the frequency of infections in a subject. So does infusion of blood products such as, e.g., red blood cells. Infusions of red blood cells lead to a risk of formation of antibodies in the subject. When antibodies to blood type antigens have been formed transfusion of the subject are difficult as it will be increasingly difficult to find suitable types of blood.

Thus, major objectives in treatment of bleedings are to obtain haemostasis in a minimum of time, thus keeping the blood loss at a minimum.

The present invention thus provides beneficial compositions, uses and methods of treatment for treatment of bleeding episodes in subjects in need of such treatment. The compositions, uses and methods may be associated with beneficial effects such as less blood loss before haemostasis is obtained, less blood needed during surgery, blood pressure kept at an acceptable level until haemostasis is obtained, faster stabilisation of blood pressure, shorter recovery time for the treated patient, shorter rehabilitation time for the treated patient, diminished formation of haematomas or formation of smaller haematomas, including haematomas in the brain, less formation of pseudo cysts, less formation of muscle contractures, faster arrest of bleedings, reduction in the number of injections needed to stop bleeding and maintain haemostasis, reduction in the amount of coagulation protein usage for arresting bleeding and maintaining haemostasis.

The administration of a preparation of a factor VII or factor VII-related polypeptide, e.g., factor VIIa, in combination with a preparation of a factor VIII or factor VIII-related polypeptide provides a shortened clotting time compared to the clotting time when either factor VIIa or factor VIII is administered alone.

The administration of a preparation of a factor VII or factor VII-related polypeptide, e.g., factor VIIa, in combination with a preparation of a factor VIII or factor VIII-related polypeptide also provides for a reduced total amount of coagulation factor usage to arrest bleeding and maintain haemostasis in a subject in need of such treatment compared to the protein usage when either factor VIIa or factor VIII is administered alone.

The administration of a preparation of a factor VII or factor VII-related polypeptide, e.g., factor VIIa, in combination with a preparation of a factor VIII or factor VIII-related polypeptide also provides for a reduced time to obtain bleeding arrest and a reduced number of injections to maintain haemostasis compared to the situation when either factor VIIa or factor VIII is administered alone. The administration of a preparation of a factor VII or factor VII-related polypeptide, e.g., factor VIIa, in combination with a preparation of a factor VIII or factor VIII-related polypeptide will also provide for a reduced number of injections to ensure full haemostasis compared to the situation when either factor VIII or factor VIIa is administered alone.

In patients suffering from haemophilia A the present invention provides a beneficial effect of simultaneous or sequential dosing of a preparation of a factor VIII or factor VIII-related polypeptide and a preparation of a factor VII or factor VII-related polypeptide. Coagulation factor VIII substitution and a preparation of a factor VII or factor VII-related polypeptide both induce arrest of bleeding in these patient groups, but have different biochemical mechanisms of action. Simultaneous dosing increases the haemostatic effect.

The present invention provides a pharmaceutical composition comprising a combination of a preparation of a factor VII or factor VII-related polypeptide and a preparation of a factor VIII or factor VIII-related polypeptide. The composition may be in the form of a single composition or it may be in the form of a multi-component kit (kit-of-parts). The composition according to the present invention is useful as a therapeutic and prophylactic procoagulant in mammals, including primates such as humans. The present invention further provides a method for treating (including prophylactically treating or preventing) bleeding episodes in a subject, including a human being.

Whenever, a first or second or third, etc., unit dose is mentioned throughout this specification this does not indicate the preferred order of administration, but is merely done for convenience purposes.

A combination of a preparation of a factor VII or factor VII-related polypeptide and a preparation of a factor VIII or factor VIII-related polypeptide is an advantageous product ensuring short clotting times and rapid formation of haemostatic plugs. The present inventors will show that a combination of factor VIIa and factor VIII can shorten the clotting time of normal human plasma more effectively than factor VIIa or factor VIII alone. Thus, by shortening the clotting time a more effective treatment of bleedings in subjects can be obtained. Moreover, patients may be treated with lower total amounts of factor VII and factor VIII or factor VIII-related polypeptides.

Factor VII Polypeptides:

In practicing the present invention, any factor VII polypeptide may be used that is effective in preventing or treating bleeding. This includes factor VII polypeptides derived from blood or plasma, or produced by recombinant means.

The present invention encompasses factor VII polypeptides, such as, e.g., those having the amino acid sequence disclosed in U.S. Patent No. 4,784,950 (wild-type human factor VII). In some embodiments, the factor VII polypeptide is human factor VIIa, as disclosed, e.g., in U.S. Patent No. 4,784,950 (wild-type factor VII). In one series of embodiments, factor VII polypeptides include polypeptides that exhibit at least about 10%, preferably at least about 30%, more preferably at least about 50%, and most preferably at least about 70%, of the specific biological activity of human factor VIIa. In one series of embodiments, factor VII polypeptides include polypeptides that exhibit at least about 90%, preferably at least about 100%, preferably at least about 120%, more preferably at least about 140%, and most preferably at least about 160%, of the specific biological activity of human factor VIIa. In one series of embodiments, factor VII polypeptides include polypeptides that exhibit at least about 70 %, preferably at least about 80 %, more preferably at least about 90 %, and most preferable at least about 95 %, of identity with the sequence of wild-type factor VII as disclosed in U.S. Patent No. 4,784,950.

As used herein, "factor VII polypeptide" encompasses, without limitation, factor VII, as well as factor VII-related polypeptides. The term "factor VII" is intended to encompass, without limitation, polypeptides having the amino acid sequence 1-406 of wild-type human factor VII (as disclosed in U.S. Patent No. 4,784,950), as well as wild-type factor VII derived from other species, such as, e.g., bovine, porcine, canine, murine, and salmon factor VII, said factor VII derived from blood or plasma, or produced by recombinant means. It further encompasses natural allelic variations of factor VII that may exist and occur from one individual to another. Also, degree and location of glycosylation or other post-translation modifications may vary depending on the chosen host cells and the nature of the host cellular environment. The term "Factor VII" is also intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa.

"Factor VII-related polypeptides" include, without limitation, factor VII polypeptides that have either been chemically modified relative to human factor VII and/or contain one or more amino acid sequence alterations relative to human factor VII (i.e., factor VII variants), and/or contain truncated amino acid sequences relative to human factor VII (i.e., factor VII fragments). Such factor VII-related polypeptides may exhibit different properties relative to human factor VII, including stability, phospholipid binding, altered specific activity, and the like.

The term "factor VII-related polypeptides" are intended to encompass such polypeptides in their uncleaved (zymogen) form, as well as those that have been proteolytically processed to yield their respective bioactive forms, which may be designated "factor VIIa-related polypeptides" or "activated factor VII-related polypeptides"

As used herein, "factor VII-related polypeptides" encompasses, without limitation, polypeptides exhibiting substantially the same or improved biological activity relative to wild-type human factor VII, as well as polypeptides in which the factor VIIa biological activity has been substantially modified or reduced relative to the activity of wild-type human factor VIIa. These polypeptides include, without limitation, factor VII or factor VIIa that has been chemically modified and factor VII variants into which specific amino acid sequence alterations have been introduced that modify or disrupt the bioactivity of the polypeptide.

It further encompasses polypeptides with a slightly modified amino acid sequence, for instance, polypeptides having a modified N-terminal end including N-terminal amino acid deletions or additions, and/or polypeptides that have been chemically modified relative to human factor VIIa.

Factor VII-related polypeptides, including variants of factor VII, whether exhibiting substantially the same or better bioactivity than wild-type factor VII, or, alternatively, exhibiting substantially modified or reduced bioactivity relative to wild-type factor VII, include, without limitation, polypeptides having an amino acid sequence that differs from the sequence of wild-type factor VII by insertion, deletion, or substitution of one or more amino acids.

Factor VII-related polypeptides, including variants, encompass those that exhibit at least about 10%, at least about 20%, at least about 25%, at least about 30%, at least about 40%, at least about 50%, at least about 60%, at least about 70%, at least about 75%, at least about 80%, at least about 90%, at least about 100%, at least about 110%, at least about 120%, or at least about 130%, of the specific activity of wild-type factor VIIa that has been produced in the same cell type, when tested in one or more of a clotting assay, proteolysis assay, or TF binding assay as described above.

Factor VII-related polypeptides, including variants, having substantially the same or improved biological activity relative to wild-type factor VIIa encompass those that exhibit at least about 25%, preferably at least about 50%, more preferably at least about 75%, more preferably at least about 100%, more preferably at least about 110%, more preferably at least about 120%, and most preferably at least about 130% of the specific activity of wild-type factor VIIa that has been produced in the same cell type, when tested in one or more of a clotting assay, proteolysis assay, or TF binding assay as described above.

Factor VII-related polypeptides, including variants, having substantially reduced biological activity relative to wild-type factor VIIa are those that exhibit less than about 25%, preferably less than about 10%, more preferably less than about 5% and most preferably less than about 1% of the specific activity of wild-type factor VIIa that has been produced in the same cell type when tested in one or more of a clotting assay, proteolysis assay, or TF binding assay as described above. Factor VII variants having a substantially modified biological activity relative to wild-type factor VII include, without limitation, factor VII variants that exhibit TF-independent Factor X proteolytic activity and those that bind TF but do not cleave Factor X.

In some embodiments the factor VII polypeptides are factor VII-related polypeptides, in particular variants, wherein the ratio between the activity of said factor VII polypeptide and the activity of native human factor VIIa (wild-type FVIIa) is at least about 1.25 when tested in the "In Vitro Hydrolysis Assay" (see "Assays", below); in other embodiments, the ratio is at least about 2.0; in further embodiments, the ratio is at least about 4.0. In some embodiments of the invention, the factor VII polypeptides are factor VII-related polypeptides, in particular variants, wherein the ratio between the activity of said factor VII polypeptide and the activity of native human factor VIIa (wild-type FVIIa) is at least about 1.25 when tested in the "In Vitro Proteolysis Assay" (see "Assays", below); in other embodiments, the ratio is at least about 2.0; in further embodiments, the ratio is at least about 4.0; in further embodiments, the ratio is at least about 8.0.

In some embodiments, the factor VII polypeptide is human factor VII, as disclosed, e.g., in U.S. Patent No. 4,784,950 (wild-type factor VII). In some embodiments, the factor VII polypeptide is human factor VIIa. In one series of embodiments, the factor VII polypeptides are factor VII-related polypeptides that exhibits at least about 10%, preferably at least about 30%, more preferably at least about 50%, and most preferably at least about 70%, of the specific biological activity of human factor VIIa. In some embodiments, the factor VII polypeptides have an amino acid sequence that differs from the sequence of wild-type factor VII by insertion, deletion, or substitution of one or more amino acids.

Non-limiting examples of factor VII variants having substantially the same or improved biological activity as wild-type factor VII include S52A-FVII, S60A-FVII (lino et al., Arch. Biochem. Biophys. 352: 182-192, 1998); L305V-FVII, L305V/M306D/D309S-FVII, L3051-FVII, L305T-FVII, F374P-FVII, V158T/M298Q-FVII, V158D/E296V/M298Q-FVII, K337A-FVII, M298Q-FVII, V158D/M298Q-FVII, L305V/K337A-FVII, V158D/E296V/M298Q/L305V-FVII, V158D/E296V/M298Q/K337A-FVII, V158D/E296V/M298Q/L305V/K337A-FVII, K157A-FVII, E296V-FVII, E296V/M298Q-FVII, V158D/E296V-FVII, V158D/M298K-FVII, and S336G-FVII; FVIIa variants exhibiting increased proteolytic stability as disclosed in U.S. Patent No. 5,580,560; factor VIIa that has been proteolytically cleaved between residues 290 and 291 or between residues 315 and 316 (Mollerup et al., Biotechnol. Bioeng. 48:501-505, 1995); and oxidized forms of factor VIIa (Kornfelt et al., Arch. Biochem. Biophys. 363:43-54, 1999). Non-limiting examples of factor VII variants having substantially reduced or modified biological activity relative to wild-type factor VII include R152E-FVIIa (Wildgoose et al., Biochem 29:3413-3420, 1990), S344A-FVIIa (Kazama et al., J. Biol. Chem. 270:66-72, 1995), FFR-FVIIa (Holst et al., Eur. J. Vasc. Endovasc. Surg. 15:515-520, 1998), and factor VIIa lacking the Gla domain, (Nicolaisen et al., FEBS Letts. 317:245-249, 1993). Non-limiting examples of chemically modified factor VII polypeptides and sequence variants are described, e.g., in U.S. Patent No. 5,997,864.

The biological activity of factor VIIa in blood clotting derives from its ability to (i) bind to tissue factor (TF) and (ii) catalyze the proteolytic cleavage of Factor IX or Factor X to produce activated Factor IX or X (Factor IXa or Xa, respectively).

For purposes of the invention, biological activity of factor VII polypeptides ("factor VII biological activity") may be quantified by measuring the ability of a preparation to promote blood clotting using factor VII-deficient plasma and thromboplastin, as described, e.g., in U.S. Patent No. 5,997,864. In this assay, biological activity is expressed as the reduction in clotting time relative to a control sample and is converted to "factor VII units" by comparison with a pooled human serum standard containing 1 unit/ml factor VII activity. Alternatively, factor VIIa biological activity may be quantified by

(i) Measuring the ability of factor VIIa or a factor VIIa -related polypeptide to produce activated Factor X (Factor Xa) in a system comprising TF embedded in a lipid membrane and Factor X. (Persson et al., J. Biol. Chem. 272: 19919-19924, 1997);
(ii) Measuring Factor X hydrolysis in an aqueous system ("In Vitro Proteolysis Assay", see below);
(iii) Measuring the physical binding of factor VIIa or a factor VIIa -related polypeptide to TF using an instrument based on surface plasmon resonance (Persson, FEBS Letts. 413:359-363, 1997); and
(iv) Measuring hydrolysis of a synthetic substrate by factor VIIa and/or a factor VIIa -related polypeptide ("In Vitro Hydrolysis Assay", see below); and
(v) Measuring generation of thrombin in a TF-independent in vitro system.

The term "factor VII biological activity" or "factor VII activity" is intended to include the ability to generate thrombin; the term also includes the ability to generate thrombin on the surface of activated platelets in the absence of tissue factor.

A factor VIIa preparation that may be used according to the invention is, without limitation, NovoSeven® (Novo Nordisk A/S, Bagsvaerd, Denmark).

Factor VIII polypeptides:

The present invention encompasses factor VIII polypeptides, such as, e.g., those having the amino acid sequence disclosed in, e.g., Toole et al.; Nature 1984; 312: 342-347 (wild-type human factor VIII).

In practicing the present invention, any factor VIII polypeptide may be used that is effective in preventing or treating bleeding. This includes factor VIII polypeptides derived from blood or plasma, or produced by recombinant means.

As used herein, "factor VIII polypeptide" encompasses, without limitation, factor VIII, as well as factor VIII-related polypeptides. The term "Factor VIII" is intended to encompass, without limitation, polypeptides having the amino acid sequence as described in Toole et al.; Nature 1984 (see above) (wild-type human factor VIII), as well as wild-type Factor VIII derived from other species, such as, e.g., bovine, porcine, canine, murine, and salmon Factor VIII. It further encompasses natural allelic variations of Factor VIII that may exist and occur from one individual to another. Also, degree and location of glycosylation or other post-translation modifications may vary depending on the chosen host cells and the nature of the host cellular environment. The term "Factor VIII" is also intended to encompass Factor VIII polypeptides in their uncleaved (zymogen) form, as well as those that have been proteolytically processed to yield their respective bioactive forms, which may be designated Factor VIIIa.

"Factor VIII-related polypeptides" include, without limitation, factor VIII polypeptides that have either been chemically modified relative to human factor VIII and/or contain one or more amino acid sequence alterations relative to human factor VIII (i.e., factor VIII variants), and/or contain truncated amino acid sequences relative to human factor VIII (i.e., factor VIII fragments). Such factor VIII-related polypeptides may exhibit different properties relative to human factor VIII, including stability, phospholipid binding, altered specific activity, and the like.
The term "factor VIII-related polypeptides" are intended to encompass such polypeptides in their uncleaved (zymogen) form, as well as those that have been proteolytically processed to yield their respective bioactive forms, which may be designated "factor VIIIa-related polypeptides" or "activated factor VIII-related polypeptides".

As used herein, "factor VIII-related polypeptides" encompasses, without limitation, polypeptides exhibiting substantially the same or improved biological activity relative to wild-type human factor VIII, as well as polypeptides, in which the factor VIII biological activity has been substantially modified or reduced relative to the activity of wild-type human factor VIII. These polypeptides include, without limitation, factor VIII or factor VIIIa that has been chemically modified and factor VIII variants into which specific amino acid sequence alterations have been introduced that modify or disrupt the bioactivity of the polypeptide.

It further encompasses polypeptides with a slightly modified amino acid sequence, for instance, polypeptides having a modified N-terminal end including N-terminal amino acid deletions or additions, and/or polypeptides that have been chemically modified relative to human factor VIII.

Factor VIII-related polypeptides, including variants of factor VIII, whether exhibiting substantially the same or better bioactivity than wild-type factor VIII, or, alternatively, exhibiting substantially modified or reduced bioactivity relative to wild-type factor VIII, include, without limitation, polypeptides having an amino acid sequence that differs from the sequence of wild-type factor VIII by insertion, deletion, or substitution of one or more amino acids.

Factor VIII-related polypeptides, including variants, encompass those that exhibit at least about 10%, at least about 20%, at least about 30%, at least about 40%, at least about 50%, at least about 60%, at least about 70%, at least about 80%, at least about 90%, at least about 100%, at least about 110%, at least about 120%, and at least about 130%, of the specific activity of wild-type factor VIII that has been produced in the same cell type, when tested in the factor VIII activity assay as described in the present specification.

Factor VIII-related polypeptides, including variants, having substantially the same or improved biological activity relative to wild-type factor VIII encompass those that exhibit at least about 25%, preferably at least about 50%, more preferably at least about 75%, more preferably at least about 100%, more preferably at least about 110%, more preferably at least about 120%, and most preferably at least about 130% of the specific biological activity of wild-type human factor VIII that has been produced in the same cell type when tested in one or more of the specific factor VIII activity assay as described. For purposes of the invention, factor VIII biological activity may be quantified as described later in the present description ("assay part").

Factor VIII-related polypeptides, including variants, having substantially reduced biological activity relative to wild-type factor VIII are those that exhibit less than about 25%, preferably less than about 10%, more preferably less than about 5% and most preferably less than about 1 % of the specific activity of wild-type factor VIII that has been produced in the same cell type when tested in one or more of the specific factor VIII activity assays as described above.

Non-limiting examples of factor VIII polypeptides include plasma-derived human factor VIII as described, e.g., in Fulcher et al.; Proc. Acad. Nat. Sci. USA 1982; 79:1648-1652, and Rotblat et al.; Biochemistry 1985; 24:4294-4300, and plasma-derived porcine FVIII as described, e.g., in Fass et al.; Blood 1982; 59: 594-600 and Knutson et al.; Blood 1982; 59: 615-624

In some embodiments the factor VIII are factor VIII-related polypeptides wherein the ratio between the activity of said factor VIII polypeptide and the activity of native human factor VIII (wild-type factor VIII) is at least about 1.25 when tested in the " chromogenic assay" (see below); in other embodiments, the ratio is at least about 2.0; in further embodiments, the ratio is at least about 4.0.

Non-limiting examples of factor VIII sequence variants are described, e.g., in Lollar et al.; Blood 2000; 95(2): 564-568 (hybrid porcine/human FVIII polypeptides) and Lollar et al.; Blood 2001;.-97(1): 169-174.

Commercially available FVIII products (so-called replacement products) are derived from normal pooled plasma or genetically engineered mammalian cell lines. Replacement products are often classified according to final purity, defined as specific activity (international units of clotting factor activity per mg of protein, IU/mg). Intermediate products have relatively low specific activity (< 50 IU/mg) because they also contain extraneous plasma proteins, such as fibrinogen, fibronectin and other non-coagulant proteins. High purity (>50 IU/mg) and ultra high purity (> 3000 IU/mg) contain little or virtual no other plasma proteins other that albumin added as a stabiliser.

Non-limiting examples of commercially available factor VIII products (concentrates and preparations) that may be used according to the present invention are, for example, without limitation, ReFacto (B-domain deleted rFVIII) from AHP/Genetics Institute, Alphanate (FVIII) from Alpha, Bioclate (rFVIII) from Aventis, Monoclate-P (factor VIII:C) from Aventis, Helixate (rFVIII) from Aventis, Recombinate (rFVIII) from Baxter, Hemofil M (FVIII) from Baxter, Kogenate (rFVIII) from Bayer, Nordiate (FVIII) from HemaSure, FACTEUR VIII-LFB (human plasma-based FVIII) from Laboratoire Francais du Fractionnement et des Biotechnologies (LFB), Hyate:C (porcine FVIII) from Speywood, and Kogenate SF (sucrose formulated rFVIII) from Bayer.

Non.limiting examples of high and ultra high activity products are Alphanate (Alpha) (low); ReFacto (AHP/ Genetics Institute), Kogenate SF (Bayer), Kogenate (Bayer), Helixate (Aventis), Recombinate (Baxter), Monoclate-P (Aventis), Hemofil M (Baxter) (all ultra high).

Definitions:

In the present context the three-letter or one-letter indications of the amino acids have been used in their conventional meaning as indicated in table 1. Unless indicated explicitly, the amino acids mentioned herein are L-amino acids. It is to be understood, that the first letter in, for example, K337 represent the amino acid naturally present at the indicated position wild-type factor VII, and that, for example, K337A-FVIIa designates the FVII-variant wherein the amino acid represented by the one-letter code K naturally present in the indicated position is replaced by the amino acid represented by the one-letter code A.

Table 1: Abbreviations for amino acids:

| Amino acid | Tree-letter code | One-lefter code |
|---|---|---|
| Glycine | Gly | G |
| Proline | Pro | P |
| Alanine | Ala | A |
| Valine | Val | V |
| Leucine | Leu | L |
| Isoleucine | Ile | I |
| Methionine | Met | M |
| Cysteine | Cys | C |
| Phenylalanine | Phe | F |
| Tyrosine | Tyr | Y |
| Tryptophan | Trp | W |
| Histidine | His | H |
| Lysine | Lys | K |
| Arginine | Arg | R |
| Glutamine | Gln | Q |
| Asparagine | Asn | N |
| Glutamic Acid | Glu | E |
| Aspartic Acid | Asp | D |

The terms "factor VII", "Factor VII" or "FVII" may be used interchangeably. The terms "factor VIIa", "Factor VIIa" or "FVIIa" may be used interchangeably. The terms "factor VIII" or "Factor VIII" or "FVIII" may be used interchangeably.

In this context, "subjects with an impaired thrombin generation" means subjects who cannot generate a full thrombin burst on the activated platelet surface and includes subjects having a generation of thrombin less that the thrombin-generation in subjects having a fully functioning, normal haemostatic system, including a normal amount and function of coagulation factors, platelets and fibrinogen, and includes, without limitations, subjects lacking factor VIII; subjects with a lowered number of platelets or platelets with a defective function (e.g., thrombocytopenia or thrombasthenia Glanzmann or subjects with excessive bleeds); subjects having lowered levels of prothrombin, FX or FVII; subjects having a lowered level of several coagulation factors (e.g., due to exessive bleeding as a consequence of trauma or extensive surgery); and subjects with lowered plasma concentrations of fibrinogen (e.g., multitransfused subjects).

The term "enhancement of the haemostatic system" means an enhancement of the ability to generate thrombin. The term "enhancing haemostasis" is intended to encompass the situations when the measured thrombin generation for a test sample containing a preparation of a factor VII or factor VII-related polypeptide and a preparation of a factor VIII or factor VIII-related polypeptide is prolonged relative to the individual thrombin generation of a control sample containing only the factor VII or factor VII-related polypeptide or the factor VIII or factor VIII-related polypeptide, respectively, when tested in the same thrombin generation assay. The thrombin generation may be assayed as described in the thrombin generation assay of the present description (see "assay part").

Clot lysis time, clot strength, fibrin clot formation, and clotting time are clinical parameters used for assaying the status of patient's haemostatic system. Blood samples are drawn from the patient at suitable intervals and one or more of the parameters are assayed by means of, e.g., thromboelastograpy as described by, e.g., Meh et al.,Blood Coagulation & Fibrinolysis 2001;12:627-637; Vig et al., Hematology, Vol. 6 (3) pp. 205-213 (2001); Vig et al., Blood coagulation & fibrinolysis, Vol. 12 (7) pp. 555-561 (2001) Oct; Glidden et al., Clinical and applied thrombosis/hemostasis, Vol. 6 (4) pp. 226-233 (2000) Oct; McKenzie et al., Cardiology, Vol. 92 (4) pp. 240-247 (1999) Apr; or Davis et al., Journal of the American Society of Nephrology, Vol. 6 (4) pp. 1250-1255 (1995).

The term "prolonging clot lysis time" is intended to encompass the situations when the measured clot lysis time for a test sample containing a preparation of a factor VII or factor VII-related polypeptide and a preparation of a factor VIII or factor VIII-related polypeptide is prolonged relative to the individual clot lysis time of a control sample containing only the factor VII or factor VII-related polypeptide or the factor VIII or factor VIII-related polypeptide, respectively, when tested in the same clot lysis assay. The clot lysis time may be assayed as described above.

The term "increasing clot strength" is intended to encompass the situations when the measured clot strength, e.g., mechanical strength, for a test sample containing a preparation of a factor VII or factor VII-related polypeptide and a preparation of a factor VIII or factor VIII-related polypeptide is increased relative to the individual clot lysis time of a control sample containing only the factor VII or factor VII-related polypeptide or the factor VIII or factor VIII-related polypeptide, respectively, when tested in the same clot strength assay. The clot strength may be assayed as described, e.g. in Carr et al, 1991. (Carr ME, Zekert SL. Measurement of platelet-mediated force development during plasma clot formation. AM J MED SCI 1991; 302: 13-8), or as described above by means of thromboelastography.

The term "enhancing fibrin clot formation" is intended to encompass the situations when the measured rate for or degree of fibrin clot formation for a test sample containing a preparation of a factor VII or factor VII-related polypeptide and a preparation of a preparation of a factor VIII or factor VIII-related polypeptide is increased relative to the individual rate for or degree of fibrin clot formation of a control sample containing only the factor VII or factor VII-related polypeptide or the factor VIII or factor VIII-related polypeptide, respectively, when tested in the same clotting assay. The fibrin clot formation may be assayed as described above.

The term "shortening clotting time" is intended to encompass the situations when the measured time for clot formation (clotting time) for a test sample containing a preparation of a factor VII or factor VII-related polypeptide and a preparation of a preparation of a factor VIII or factor VIII-related polypeptide is increased relative to the individual clotting time of a control sample containing only the factor VII or factor VII-related polypeptide or the factor VIII or factor VIII-related polypeptide respectively, when tested in the same clotting assay. The clotting time may be assayed by means of standard PT og aPTT assays, which are known to the general skilled person.

As used herein the term "bleeding disorder" reflects any defect, congenital, acquired or induced, of cellular or molecular origin that is manifested in bleeding episodes. Examples of bleeding disorders include, but are not limited to, clotting factor deficiencies (e.g. deficiency of coagulation factors VIII, iX, XI or VII), clotting factor inhibitors, defective platelet function (e.g., Glanzmann thombasthenia and Bernard-Soulier syndrome), thrombocytopenia, von Willebrand's disease, and coagulophathy such as that caused by a dilution of coagulation proteins, increased fibrinolysis and lowered number of platelets due to bleedings and/or transfusions (e.g., in multi transfused subjects having been subjected to surgery or trauma).

Bleeding refers to extravasation of blood from any component of the circulatory system. The term "bleeding episodes" is meant to include unwanted, uncontrolled and often excessive bleeding in connection with surgery, trauma, or other forms of tissue damage, as well as unwanted bleedings in subjects having bleeding disorders. Bleeding episodes may occur in subjects having a basically normal coagulation system but experiencing a (temporary) coagulophathy, as well as in subjects having congenital or acquired coagulation or bleeding disorders. In subjects having a defective platelet function, the bleedings may be likened to bleedings caused by haemophilia because the haemostatic system, as in haemophilia, lacks or has abnormal essential clotting "compounds" (e.g., platelets or von Willebrand factor protein). In subjects who experience extensive tissue damage, for example in association with surgery or vast trauma, the normal haemostatic mechanism may be overwhelmed by the demand of immediate haemostasis and they may develop excessive bleeding in spite of a basically (pre-trauma or pre-surgery) normal haemostatic mechanism. Such subjects, who further often are multi transfused, develop a (temporary) coagulopathy as a result of the bleeding and/or transfusions (i.e., a dilution of coagulation proteins, increased fibrinolysis and lowered number of platelets due to the bleeding and/or transfusions). Bleedings may also occur in organs such as the brain, inner ear region and eyes; these are areas with limited possibilities for surgical haemostasis and thus problems with achieving satisfactory haemostasis. Similar problems may arise in the process of taking biopsies from various organs (liver, lung, tumour tissue, gastrointestinal tract) as well as in laparoscopic surgery and radical retropubic prostatectomy. Common for all these situations is the difficulty to provide haemostasis by surgical techniques (sutures, clips, etc.) which also is the case when bleeding is diffuse (e.g., haemorrhagic gastritis and profuse uterine bleeding). Bleedings may also occur in subjects on anticoagulant therapy in whom a defective haemostasis has been induced by the therapy given; these bleedings are often acute and profuse. Anticoagulant therapy is often given to prevent thromboembolic disease. Such therapy may include heparin, other forms of proteoglycans, warfarin or other forms of vitamin K-antagonists as well as aspirin and other platelet aggregation inhibitors, such as, e.g., antibodies or other inhibitors of GP IIb/IIIa activity. Bleeding episodes are also meant to include, without limitation, uncontrolled and excessive bleeding in connection with surgery or trauma in subjects having acute haemarthroses (bleedings in joints), chronic haemophilic arthropathy, haematomas, (e.g., muscular, retroperitoneal, sublingual and retropharyngeal), bleedings in other tissue, haematuria (bleeding from the renal tract), cerebral haemorrhage, surgery (e.g., hepatectomy), dental extraction, and gastrointestinal bleedings (e.g., UGI bleeds). The bleeding episodes may be associated with inhibitors against factor VIII; haemophilia A; haemophilia A with inhibitors; haemophilia B; deficiency of factor VII; deficiency of Factor XI; thrombocytopenia; deficiency of von Willebrand factor (von Willebrand's disease); severe tissue damage; severe trauma; surgery; laparoscopic surgery; haemorrhagic gastritis; taking biopsies; anticoagulant therapy; upper gastroentestinal bleedings (UGI); or stem cell transplantation. The bleeding episodes may be profuse uterine bleeding; occurring in organs with a limited possibility for mechanical haemostasis; occurring in the brain; occurring in the inner ear region; or occurring in the eyes. The terms "bleeding episodes" and "bleedings" may, where appropriate, be used interchangeably.

In this context, the term "treatment" is meant to include both prevention of an expected bleeding, such as, for example, in surgery, and regulation of an already occurring bleeding, such as, for example, in trauma, with the purpose of inhibiting or minimising the bleeding. The above-referenced "expected bleeding" may be a bleeding expected to occur in a particular tissue or organ, or it may be an unspecified bleeding. Prophylactic administration of a preparation of a factor VII or factor VII-related polypeptide and a preparation of a factor VIII or factor VIII-related polypeptide is thus included in the term "treatment".

The term "subject" as used herein is intended to mean any animal, in particular mammals, such as humans, and may, where appropriate, be used interchangeably with the term "patient".

The factor VII or factor VII-related polypeptides and factor VIII or factor VIII-related polypeptides as defined in the present specification may be administered simultaneously or sequentially. The factors may be supplied in single-dosage form wherein the single-dosage form contains both coagulation factors, or in the form of a kit-of-parts comprising a preparation of a factor VII or factor VII-related polypeptide as a first unit dosage form and a preparation of a factor VIII or factor VIII-related polypeptide as a second unit dosage form. The second unit dosage form may be in the form of a high-, medium- or low-activity factor VIII product. High-activity products are preferred. Most preferred are recombinant high-activity products. Whenever a first or second or third, etc., unit dose is mentioned throughout this specification this does not indicate the preferred order of administration, but is merely done for convenience purposes

By "simultaneous" dosing of a preparation of a factor VII or factor VII-related polypeptide and a preparation of a factor VIII or factor VIII-related polypeptide is meant administration of the coagulation factor proteins in single-dosage form, or administration of a first coagulation factor protein followed by administration of a second coagulation factor protein with a time separation of no more than 15 minutes, preferably 10, more preferred 5, more preferred 2 minutes. Either factor may be administered first.

By "sequential" dosing is meant administration of a first coagulation factor protein followed by administration of a second coagulation factor protein with a time separation of up to 2 hours, preferably from 1 to 2 hours, more preferred up to 1 hour, more preferred from 30 minutes to 1 hour, more preferred up to 30 minutes, more preferred from 15 to 30 minutes.

Either of the two unit dosage form, or coagulation factor proteins, may be administered first. Preferably, both products are injected through the same intravenous access.

By "level of factor VIII" or "factor VIII level" is meant the level of the patient's clotting factor VIII activity compared to the level in healthy subjects. The level is designated as a percentage of the normal level. The terms may, where appropriate, be used interchangeably.

By "reduced level of factor VIII" or "reduced factor VIII level" is meant a decrease in the presence or activity of Factor VIII in the blood stream compared to the mean factor VIII level in a population of subjects having no coagulation factor VIII deficiency or inhibitors to coagulation factor VIII. Based on its purification from human plasma, the concentration of factor VIII in the normal adult is about 100 to 200 ng/ml of plasma (mean value) which is equivalent to about 0.1 $\mu$M; this equivalents to 0.60 - 1.60 U/ml.

In normal healthy individuals, factor VIII activity and antigen levels vary between 60 and 160 % of normal pooled plasma. Clinically, the level of circulating factor VIII can be measured by either a coagulant or an immunologic assay. Factor VIII procoagulant activity is determined by the ability of the patient's plasma to correct the clotting time of factor VIII-deficient plasma (e.g., an APTT assay, see below; see also "assay part" of the present description).

One unit of factor VIII has been defined as the amount of factor VIII present in one millilitre of normal (pooled) human plasma (corresponding to a factor VIII level of 100 %).

One unit of factor VII is defined as the amount of factor VII present in 1 ml of normal plasma, corresponding to about 0.5 $\mu$g protein. After activation 50 units correspond to about 1 $\mu$g protein.

By "deficiency" is meant a decrease in the presence or activity of, e.g., factor VIII in plasma compared to that of normal healthy individuals. The term may, where appropriate, be used interchangeably with "reduced factor VIII level".

By "APTT" or "aPTT" is meant the activated partial thromboplastin time (described by, e.g., Proctor RR, Rapaport SI: The partial thromboplastin time with kaolin; a simple screening test for first-stage plasma clotting factor deficiencies. Am J Clin Pathol 36:212, 1961).

By "factor VIII-responsive syndrome" is meant a syndrome where exogenous factor VIII administered to the subject in need thereof may prevent, cure or ameliorate any symptoms, conditions or diseases, expected or present, caused by the syndrome. Included are, without limitation, syndromes caused by a reduced level of factor VIII, e.g., bleeding disorders such as, without limitation, haemophilia A, or syndromes caused by inhibitors to factor VIII.

By "factor VII-responsive syndrome" is meant a syndrome where exogenous factor VII, preferably factor VIIa, administered to the subject in need thereof may prevent, cure or ameliorate any symptoms, conditions or diseases, expected or present, caused by the syndrome. Included are, without limitation, syndromes caused by a reduced level of clotting factors VIII, IX, XI or VII, clotting factor inhibitors, defective platelet function (e.g., Glanzmann thombasthenia and Bernard-Soulier syndrome), thrombocytopenia, von Willebrand's disease, and coagulophathy such as that caused by a dilution of coagulation proteins, increased fibrinolysis and lowered number of platelets due to bleedings and/or transfusions (e.g., in multi transfused subjects having been subjected to surgery or trauma).

"Half-life" refers to the time required for the plasma concentration of a factor VII or factor VII-related polypeptide or a factor VIII or factor VIII-related polypeptide to decrease from a particular value to half of that value.

By "primary haemostasis" is meant the initial generation of thrombin by FXa and TF:factor VIIa, the subsequent activation of platelets and formation of the initial loose plug of activated, adhered platelets which has not yet been stabilized by fibrin and, finally, by cross-linked fibrin. If not stabilized by the fibrin formed during the second step of the haemostatic process (maintained haemostasis), the plug is easily dissolved by the fibrinolytic system

By "secondary haemostasis" or "maintained haemostasis" is meant the secondary, full, and major, burst or generation of thrombin taking place on the surface of activated platelets and catalysed by factor VIIIa and factor VIIIa, the subsequent formation of fibrin and the stabilization of the initial platelet plug. Stabilization of the plug by fibrin leads to full haemostasis.

By "full haemostasis" is meant the formation of a stable and solid fibrin clot or plug at the site of injury which effectively stops the bleeding and which is not readily dissolved by the fibrinolytic system. In this context, the term haemostasis will be used to represent full haemostasis as described above.

As used herein, a "preparation" of a coagulation factor, e.g., factor VIII, is one in which factor VIII is the predominant factor. In one embodiment the coagulation factor is present in the preparation in an amount of more than 20% (w/w) of the total amount of protein, more preferred 30%, more preferred 40%, more preferred 50%, more preferred 60%, more preferred 70%, more preferred 80%, more preferred 90%, more preferred 95%, more preferred 98%, more preferred 99%.

In a preferred embodiment, the coagulation factor is present in an amount of more than 50% (w/w) of the total amount of coagulation factor protein, more preferred 80%, more preferred 90%, more preferred 95%, more preferred 98%, more preferred 99%.

The total amount of protein in such preparation may be measured by generally known methods, e.g, by measuring optical density. Amounts of factor VIII coagulation protein may be measured by generally known methods such as standard Elisa immuno assays. In general terms, such assay is conducted by contacting a solution of the factor VIII protein- containing preparation with an anti-FVIII antibody immobilised onto the elisa plate, subsequently contacting the immobilised antibody-factor VIII complex with a second anti FVIII antibody carrying a marker, the amounts of which, in a third step, are measured. The amounts of each coagulation factor may be measured in a similar way using appropriate antibodies. The total amount of coagulation factor protein present in a preparation is determined by adding the amounts of the individual coagulation factor proteins. In one embodiment, the preparation comprises isolated coagulation factor. In another embodiment the preparation is free of coagulation factor II and coagulation factor IIa.

As used herein, the term "isolated" refers to coagulation factors, e.g., factor VIII or factor VIII-related polypeptides that have been separated from the cell in which they were synthesized or the medium in which they are found in nature (e.g., plasma or blood). Separation of polypeptides from their cell of origin may be achieved by any method known in the art, including, without limitation, removal of cell culture medium containing the desired product from an adherent cell culture; centrifugation or filtration to remove non-adherent cells; and the like. Separation of polypeptides from the medium in which they naturally occur may be achieved by any method known in the art, including, without limitation, affinity chromatography, such as, e.g., on an anti-factor VII or anti-factor VIII antibody column, respectively; hydrophobic interaction chromatography; ion-exchange chromatography; size exclusion chromatography; electrophoretic procedures (e.g., preparative isoelectric focusing (IEF)), differential solubility (e.g., ammonium sulfate precipitation), or extraction and the like.

***Abbreviations:***

| | |
|---|---|
| TF | tissue factor |
| FVII | factor VII in its single-chain, unactivated form |
| FVIIa | factor VII in its activated form |
| rFVIIa | recombinant factor VII in its activated form |
| factor VIII factor VIII | in its zymogenic, unactivated form |
| factor VIIIa | factor VIII in its activated form |
| rfactor VIII | recombinant factor VIII |
| rfactor VIIIa | recombinant factor VIIIa |

***Preparation of compounds:***

Human purified factor VIIa suitable for use in the present invention is preferably made by DNA recombinant technology, e.g. as described by Hagen et al., Proc.Natl.Acad.Sci. USA 83: 2412-2416, 1986, or as described in European Patent No. 200.421 (ZymoGenetics, Inc.).

Factor VII may also be produced by the methods described by Broze and Majerus, J.Biol.Chem. 255 (4): 1242-1247, 1980 and Hedner and Kisiel, J.Clin.Invest. 71: 1836-1841, 1983. These methods yield factor VII without detectable amounts of other blood coagulation factors. An even further purified factor VII preparation may be obtained by including an additional gel filtration as the final purification step. factor VII is then converted into activated factor VIIa by known means, e.g. by several different plasma proteins, such as factor XIIa, IX a or Xa. Afternatively, as described by Bjoern et al. (Research Disclosure, 269 September 1986, pp. 564-565), factor VII may be activated by passing it through an ion-exchange chromatography column, such as Mono Q® (Pharmacia fine Chemicals) or the like.

Factor VII -related polypeptides may produced by modification of wild-type factor VII or by recombinant technology. Factor VII -related polypeptides with altered amino acid sequence when compared to wild-type factor VII may be produced by modifying the nucleic acid sequence encoding wild-type factor VII either by altering the amino acid codons or by removal of some of the amino acid codons in the nucleic acid encoding the natural factor VII by known means, e.g. by site-specific mutagenesis.

It will be apparent to those skilled in the art that substitutions can be made outside the regions critical to the function of the factor VIIa or factor VIII-molecule and still result in an active polypeptide. Amino acid residues essential to the activity of the factor VII or factor VII-related polypeptide or factor VIII or factor VIII-related polypeptide, and therefore preferably not subject to substitution, may be identified according to procedures known in the art, such as site-directed mutagenesis or alanine-scanning mutagenesis (see, *e.g.*, Cunningham and Wells, 1989, Science 244: 1081-1085). In the latter technique, mutations are introduced at every positively charged residue in the molecule, and the resultant mutant molecules are tested for coagulant, respectively cross-linking activity to identify amino acid residues that are critical to the activity of the molecule. Sites of substrate-enzyme interaction can also be determined by analysis of the three-dimensional structure as determined by such techniques as nuclear magnetic resonance analysis, crystallography or photoaffinity labelling (see, e.g., de Vos et al., 1992, Science 255: 306-312; Smith et al., 1992, Journal of Molecular Biology 224: 899-904; Wiodaver et al., 1992, FEBS Letters 309: 59-64).

The introduction of a mutation into the nucleic acid sequence to exchange one nucleotide for another nucleotide may be accomplished by site-directed mutagenesis using any of the methods known in the art. Particularly useful is the procedure that utilizes a super coiled, double stranded DNA vector with an insert of interest and two synthetic primers containing the desired mutation. The oligonucleotide primers, each complementary to opposite strands of the vector, extend during temperature cycling by means of *Pfu* DNA polymerase. On incorporation of the primers, a mutated plasmid containing staggered nicks is generated. Following temperature cycling, the product is treated with *Dpn*I, which is specific for methylated and hemi-methylated DNA to digest the parental DNA template and to select for mutation-containing synthesized DNA. Other procedures known in the art for creating, identifying and isolating variants may also be used, such as, for example, gene shuffling or phage display techniques.

Separation of polypeptides from their cell of origin may be achieved by any method known in the art, including, without limitation, removal of cell culture medium containing the desired product from an adherent cell culture; centrifugation or filtration to remove non-adherent cells; and the like.

Optionally, factor VII or factor VII-related polypeptides may be further purified. Purification may be achieved using any method known in the art, including, without limitation, affinity chromatography, such as, e.g., on an anti-factor VII antibody column (see, e.g., Wakabayashi et al., J. Biol. Chem. 261:11097, 1986; and Thim et al., Biochem. 27:7785, 1988); hydrophobic interaction chromatography; ion-exchange chromatography; size exclusion chromatography; electrophoretic procedures (e.g., preparative isoelectric focusing (IEF), differential solubility (e.g., ammonium sulfate precipitation), or extraction and the like. See, generally, Scopes, Protein Purification, Springer-Verlag, New York, 1982; and Protein Purification, J.C. Janson and Lars Ryden, editors, VCH Publishers, New York, 1989. Following purification, the preparation preferably contains less than about 10% by weight, more preferably less than about 5% and most preferably less than about 1%, of non-factor VII or factor VII-related polypeptides derived from the host cell.

Factor VII or factor VII-related polypeptides may be activated by proteolytic cleavage, using Factor XIIa or other proteases having trypsin-like specificity, such as, e.g., Factor IXa, kallikrein, Factor Xa, and thrombin. See, e.g., Osterud et al., Biochem. 11:2853 (1972); Thomas, U.S. Patent No. 4,456,591; and Hedner et al., J. Clin. Invest. 71:1836 (1983). Alternatively, factor VII or factor VII-related polypeptides may be activated by passing it through an ion-exchange chromatography column, such as Mono Q® (Pharmacia) or the like. The resulting activated factor VII or factor VII-related polypeptide may then be formulated and administered as described below.

Factor VIII for use within the present invention may be isolated from plasma according to known methods, such as those disclosed, e.g., by Fulcher et al.; Proc. Acad. Nat. Sci. USA 1982; 79:1648-1652, and Rotblat et al.; Biochemistry 1985; 24:4294-4300. It is preferred, however, to use recombinant factor VIII so as to avoid to the use of blood- or tissue-derived products that carry a risk of disease transmission. Human purified Factor VIII suitable for use in the present invention is preferably made by DNA recombinant technology, e.g. as described by U.S. Patents Nos. 4,757,006 and 4,965,199.

Factor VIII -related polypeptides may produced by modification of wild-type factor VIII or by recombinant technology. Factor VIII -related polypeptides with altered amino acid sequence when compared to wild-type factor VIII may be produced by modifying the nucleic acid sequence encoding wild-type factor VIII either by altering the amino acid codons or by removal of some of the amino acid codons in the nucleic acid encoding the natural factor VIII by known means, e.g. by site-specific mutagenesis, as described in more detail above. Separation of polypeptides from their cell of origin may be achieved by any method known in the art, including, without limitation, removal of cell culture medium containing the desired product from an adherent cell culture; centrifugation or filtration to remove non-adherent cells; and the like. Optionally, factor VIII or factor VIII-related polypeptides may be further purified. Purification may be achieved using any method known in the art, including, without limitation, affinity chromatography, such as, e.g., on an anti-factor VIII antibody column; hydrophobic interaction chromatography; ion-exchange chromatography; size exclusion chromatography; electrophoretic procedures (e.g., preparative isoelectric focusing (IEF), differential solubility (e.g., ammonium sulfate precipitation), or extraction and the like, as described in more detail above. Following purification, the preparation preferably contains less than about 10% by weight, more preferably less than about 5% and most preferably less than about 1%, of non-factor VIII or factor VIII-related polypeptides derived from the host cell. The resulting activated factor VIII or factor VIII-related polypeptide may then be formulated and administered as described below.

As will be appreciated by those skilled in the art, it is preferred to use factor VIII polypeptides and factor VII polypeptides syngeneic with the subject in order to reduce the risk of inducing an immune response. Preparation and characterization of non-human factor VIII has been disclosed by, for example, Fass et al.; Blood 1982; 59: 594-600. The present invention also encompasses the use of such factor VIII polypeptides and factor VII polypeptides within veterinary procedures.

### *Pharmaceutical Compositions and Methods of Use*

The preparations of the present invention may be used to treat any factor VIII responsive syndrome, such as, e.g., bleeding disorders, including, without limitation, those caused by clotting factor deficiencies (e.g., haemophilia A), or by (low or medium titre of) inhibitors to factor VIII.

The preparations of the present invention may be used to treat any factor VII responsive syndrome, such as, e.g., bleeding disorders, including, without limitation, syndromes caused by a reduced level of clotting factors VIII, IX, XI or VII, clotting factor inhibitors, defective platelet function (e.g., Glanzmann thombasthenia and Bernard-Soulier syndrome), thrombocytopenia, von Willebrand's disease, and coagulophathy such as that caused by a dilution of coagulation proteins, increased fibrinolysis and lowered number of platelets due to bleedings and/or transfusions (e.g., in multi transfused subjects having been subjected to surgery or trauma).

Pharmaceutical compositions comprising a preparation of a factor VII or factor VII-related polypeptide and a preparation of a factor VIII or factor VIII-related polypeptide according to the present invention are primarily intended for parenteral administration for prophylactic and/or therapeutic treatment. Preferably, the pharmaceutical compositions are administered parenterally, i.e., intravenously, subcutaneously, or intramuscularly; intravenously being most preferred. They may also be administered by continuous or pulsatile infusion.

Pharmaceutical compositions or formulations according to the invention comprise a preparation of a preparation of a factor VII or factor VII-related polypeptide, or a preparation of a preparation of a factor VIII or factor VIII-related polypeptide, or a preparation of a preparation of a factor VII or factor VII-related polypeptide in combination with a preparation of a preparation of a factor VIII or factor VIII-related polypeptide in combination with, preferably dissolved in, a pharmaceutically acceptable carrier, preferably an aqueous carrier or diluent. A variety of aqueous carriers may be used, such as water, buffered water, 0.4% saline, 0.3% glycine and the like. The preparations of the invention can also be formulated using non-aqueous carriers, such as, e.g., in the form of a gel or as liposome preparations for delivery or targeting to the sites of injury. Liposome preparations are generally described in, e.g., U.S. Patents Nos. 4,837,028, 4,501,728, and 4,975,282. The compositions may be sterilised by conventional, well-known sterilisation techniques. The resulting aqueous solutions may be packaged for use or filtered under aseptic conditions and lyophilised, the lyophilised preparation being combined with a sterile aqueous solution prior to administration.

The compositions may contain pharmaceutically acceptable auxiliary substances or adjuvants, including, without limitation, pH adjusting and buffering agents and/or tonicity adjusting agents, such as, for example, sodium acetate, sodium lactate, sodium chloride, potassium chloride, calcium chloride, etc.

Formulations may further include one or more diluents, emulsifiers, preservatives, buffers, excipients, etc. and may be provided in such forms as liquids, powders, emulsions, controlled release, etc. One skilled in this art may formulate the compositions of the invention an appropriate manner, and in accordance with accepted practices, such as those disclosed in Remington's Pharmaceutical Sciences, Gennaro, ed., Mack Publishing Co., Easton, PA, 1990. Thus, a typical pharmaceutical composition for intravenous infusion could be made up to contain 250 ml of sterile Ringer's solution and 10 mg of the preparation.

The compositions containing the preparations of the present invention can be administered for prophylactic and/or therapeutic treatments. In therapeutic applications, compositions are administered to a subject already suffering from a disease, as described above, in an amount sufficient to cure, alleviate or partially arrest the clinical manifestations of the disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix.

Local delivery of the preparations of the present invention, such as, for example, topical application, may be carried out, e.g., by means of a spray, perfusion, double balloon catheters, stent, incorporated into vascular grafts or stents, hydrogels used to coat balloon catheters, or other well established methods. In any event, the pharmaceutical compositions should provide a quantity of the preparation sufficient to effectively treat the condition.

The concentration of factor VII or factor VII-related polypeptide, factor VIII or factor VIII-related polypeptide, or factor VII or factor VII-related polypeptide in combination with factor VII or factor VIII-related polypeptide in these formulations can vary widely, i.e., from less than about 0.5% by weight, usually at or at least about 1 % by weight to as much as 15 or 20% by weight and will be selected primarily by fluid volumes, viscosities, etc., in accordance with the particular mode of administration selected. Administration by injection or infusion, in particular injection, is preferred. Thus, the factor VII or factor VII-related polypeptide and the factor VIII or factor VIII-related polypeptide are prepared in a form suitable for intravenous administration, such as a preparation that is either a dissolved lyophilized powder or a liquid formulation containing both the factor VII or factor VII-related polypeptide and the factor VIII or factor VIII-related polypeptide in one dosage form, or a dissolved lyophilized powder or a liquid formulation containing the factor VII or factor VII-related polypeptide in one dosage form and dissolved lyophilized powder or a liquid formulation containing the factor VIII or factor VIII-related polypeptide in another dosage form.

It is to be understood that the amount of factor VI or factor VII-related polypeptide and the amount of factor VIII or factor VIII-related polypeptide together comprise an aggregate effective amount for treating the bleeding episode.

It must be kept in mind that the materials of the present invention may generally be employed in serious disease or injury states, that is, life threatening or potentially life threatening situations. In such cases, in view of the minimization of extraneous substances and general lack of immunogenicity of factor VIIa and factor VIII in humans, it is possible and may be felt desirable by the treating physician to administer a substantial excess of these compositions.

In prophylactic applications, compositions containing a preparation of a factor VII or factor VII-related polypeptide and a preparation of a factor VIII or factor VIII-related polypeptide are administered to a subject susceptible to or otherwise at risk of a disease state or injury to enhance the subject's own coagulative capability. Such an amount is defined to be a "prophylactically effective dose." It is to be understood that the amount of factor VII or factor VII-related polypeptide and the amount of factor VIII or factor VIII-related polypeptide together comprise an aggregate effective amount for preventing a bleeding episode.

Single or multiple administrations of the compositions can be carried out with dose levels and patterns being selected by the treating physician. The compositions may be administered one or more times per day or week. An effective amount of such a pharmaceutical composition is the amount that provides a clinically significant effect against bleeding episodes. Such amounts will depend, in part, on the particular condition to be treated, age, weight, and general health of the subject, and other factors evident to those skilled in the art.

The composition of the invention is generally administered in a single dose before the expected bleeding or at the start of the bleeding. It may however also be given repeatedly (in multiple doses) preferably with intervals of 2-4-6-12 hour, depending on the dose given and the condition of the subject.

For treatment in connection with deliberate interventions, the factor VII or factor VII-related polypeptide and the factor VIII or factor VIII-related polypeptide will typically be administered within about 24 hours prior to performing the intervention, and for as much as 7 days or more thereafter. Administration as a coagulant can be by a variety of routes as described herein.

The composition may be in the form of a single preparation (single-dosage form) comprising both a preparation of a preparation of a factor VII or factor VII-related polypeptide and a preparation of a preparation of a factor VIII or factor VIII-related polypeptide in suitable concentrations. The composition may also be in the form of a kit-of-parts consisting of a first unit dosage form comprising a preparation of a preparation of a factor VII or factor VII-related polypeptide and a second unit dosage form comprising a preparation of a preparation of a factor VIII or factor VIII-related polypeptide. In this case, the factor VII or factor VII-related polypeptide and the factor VIII or factor VIII-related polypeptide should be administered one after the other, preferably within about 15 minutes of each other, for example within 10 minutes of each other or, preferably, within 5 minutes or, more preferred, within 2 minutes of each other. Either of the two unit dosage forms can be administered first.

The kit includes at least two separate pharmaceutical compositions. The kit includes container means for containing the separate compositions such as a divided bottle or a divided foil packet. Typically the kit includes directions for the administration of the separate components. The kit form is particularly advantageous when the separate components are preferably administered in different dosage forms, are administered at different dosage intervals, or when titration of the individual components of the combination is desired by the prescribing physician.

The amount of factor VII or factor VII-related polypeptide and the amount of factor VIII or factor VIII-related polypeptide administered according to the present invention may vary from a ratio of between about 1:100 to about 100:1 (w/w). The ratio of factor VII to factor VIII may thus be, e.g., about 1:100, or 1:90, or 1:80, or 1:70 or 1:60, or 1:50, or 1:40, or 1:30, or 1:20, or 1:10, or 1:5, or 1:2, or 1:1, or 2:1, or 5:1, or 10:1, or 20:1, or 30.1, or 40:1, or 50:1, or 60:1, or 70:1, or 80:1, or 90:1, or 100:1; or between about 1:90 to about 1:1, or between about 1:80 to about 1:2, or between about 1:70 to about 1:5, or between about 1:60 to about 1:10, or between about 1:50 to about 1:25, or between about 1:40 to about 1:30, or between about 90:1 to about 1:1, or between about 80:1 to about 2:1, or between about 70:1 to about 5:1, or between about 60:1 to about 10:1, or between about 50:1 to about 25:1, or between about 40:1 to about 30:1.

The dose of the factor VII or factor VII-related polypeptide ranges from what corresponds to about 0.05 mg to about 500 mg/day of wild-type factor VII, e.g., from about 1 mg to about 200 mg/day, or, e.g., from about 5 mg to about 175 mg/day for a 70-kg subject as loading and maintenance doses, depending on the weight of the subject, the condition and the severity of the condition.

The dose of the factor VIII or factor VIII-related polypeptide ranges from what corresponds to about 0.05 mg to about 500 mg/day of wild-type factor VIII, e.g., from about 1 mg to about 200 mg/day, or, e.g., from about 1 mg to about 175 mg/day for a 70-kg subject as loading and maintenance doses, depending on the weight of the subject, the condition and the severity of the condition.

When treating subjects with a reduced level of factor VIII, the below doses are preferred:

When dosing of a factor VIII or factor VIII-related polypeptide to plasma activity level up to 10% of normal factor VIII activity:

Preferred factor VII or factor VII-related polypeptide levels: 15-300 microgram/kg b.w.
More preferred factor VII or factor VII-related polypeptide levels: 30-250 microgram/kg b.w.
Most preferred factor VII or factor VII-related polypeptide levels: 60-180 microgram/kg b.w.

When dosing of a factor VIII or factor VIII-related polypeptide to a plasma activity level up to 30% of normal factor VIII activity
Preferred factor VII or factor VII-related polypeptide levels: 15 - 300 microgram/kg b.w.
More preferred factor VII or factor VII-related polypeptide levels: 30 - 250 microgram/kg b.w.
Most preferred factor VII or factor VII-related polypeptide levels: 60 - 180 microgram/kg b.w.

When dosing of a factor VIII or factor VIII-related polypeptide to a plasma activity level up to 50 % of normal factor VIII activity
Preferred factor VII or factor VII-related polypeptide levels: 15 - 300 microgram/kg b.w.;
More preferred factor VII or factor VII-related polypeptide levels: 30 - 250 microgram/kg b.w.;
Most preferred factor VII or factor VII-related polypeptide levels: 60 - 180 microgram/kg b.w.

When dosing of a factor VIII or factor VIII-related polypeptide to a plasma activity level up to 80% of normal factor VIII activity:

Preferred factor VII or factor VII-related polypeptide levels: 5 - 300 microgram/kg b.w.;
More preferred factor VII or factor VII-related polypeptide levels: 10 - 180 microgram/kg b.w.;
More preferred factor VII or factor VII-related polypeptide levels: 30 - 120 microgram/kg b.w.;
Most preferred factor VII or factor VII-related polypeptide levels: 60 - 120 microgr/kg b.w.

When dosing of a factor VIII or factor VIII-related polypeptide to a plasma activity level up to 100% of normal factor VIII activity:

Preferred factor VII or factor VII-related polypeptide levels: 5 - 300 microgram/kg b.w.;
More preferred factor VII or factor VII-related polypeptide levels: 10 - 180 microgram/kg b.w.
Most preferred factor VII or factor VII-related polypeptide levels: 60 - 120 microgram/kg b.w.

Dosing can be calculated by assuming that 1 unit per kg of b.w. of FVIII replacement raises the plasma activity by approx. 0.02 U per ml (2%). The patient's factor VIII level is monitored by drawing blood samples at suitable intervals and analysing for factor VIII activity (see specification above).

*Assays:*

Test for factor VIIa activity:

A suitable assay for testing for factor VIIa activity and thereby selecting suitable factor VIIa variants can be performed as a simple preliminary *in vitro* test:

*In Vitro* Hydrolysis Assay

Native (wild-type) factor VIIa and factor VIIa variant (both hereafter referred to as "factor VIIa") may be assayed for specific activities. They may also be assayed in parallel to directly compare their specific activities. The assay is carried out in a microtiter plate (MaxiSorp, Nunc, Denmark). The chromogenic substrate D-Ile-Pro-Arg-*p*-nitroanilide (S-2288, Chromogenix, Sweden), final concentration 1 mM, is added to factor VIIa (final concentration 100 nM) in 50 mM Hepes, pH 7.4, containing 0.1 M NaCl, 5 mM $CaCl_2$ and 1 mg/ml bovine serum albumin. The absorbance at 405 nm is measured continuously in a SpectraMax™ 340 plate reader (Molecular Devices, USA). The absorbance developed during a 20-minute incubation, after subtraction of the absorbance in a blank well containing no enzyme, is used to calculate the ratio between the activities of variant and wild-type factor VIIa:

$$\text{Ratio} = (A_{405\ nm}\ \text{factor VIIa variant})/(A_{405\ nm}\ \text{factor VIIa wild-type}).$$

Based thereon, factor VIIa variants with an activity comparable to or higher than native factor VIIa may be identified, such as, for example, variants where the ratio between the activity of the variant and the activity of native factor VII (wild-type FVII) is around, versus above 1.0.

The activity of factor VIIa or factor VIIa variants may also be measured using a physiological substrate such as factor X, suitably at a concentration of 100-1000 nM, where the factor Xa generated is measured after the addition of a suitable chromogenic substrate (eg. S-2765). In addition, the activity assay may be run at physiological temperature.

*In Vitro Proteolysis Assay*

Native (wild-type) factor VIIa and factor VIIa variant (both hereafter referred to as "factor VIIa") are assayed in parallel to directly compare their specific activities. The assay is carried out in a microtiter plate (MaxiSorp, Nunc, Denmark). Factor VIIa (10 nM) and Factor X (0.8 microM) in 100 microL 50 mM Hepes, pH 7.4, containing 0.1 M NaCl, 5 mM CaCl2 and 1 mg/ml bovine serum albumin, are incubated for 15 min. Factor X cleavage is then stopped by the addition of 50 microL 50 mM Hepes, pH 7.4, containing 0.1 M NaCl, 20 mM EDTA and 1 mg/ml bovine serum albumin. The amount of Factor Xa generated is measured by addition of the chromogenic substrate Z-D-Arg-Gly-Arg-p-nitroanilide (S-2765, Chromogenix, Sweden), final concentration 0.5 mM. The absorbance at 405 nm is measured continuously in a Spectra-Max™ 340 plate reader (Molecular Devices, USA). The absorbance developed during 10 minutes, after subtraction of the absorbance in a blank well containing no FVIIa, is used to calculate the ratio between the proteolytic activities of variant and wild-type factor VIIa:

$$\text{Ratio} = (A405\ nm\ \text{factor VIIa variant})/(A405\ nm\ \text{factor VIIa wild-type}).$$

Based thereon, factor VIIa variants with an activity comparable to or higher than native factor VIIa may be identified, such as, for example, variants where the ratio between the activity of the variant and the activity of native factor VII (wild-type FVII) is around, versus above 1.0.

Thrombin generation assay:

The ability of factor VII or factor VII-related polypeptides or factor VIII or factor VIII-related polypeptides (e.g., variants) to generate thrombin can be measured in an assay comprising all relevant coagulation factors and inhibitors at physiological concentrations and activated platelets (as described on p. 543 in Monroe et al. (1997) Brit. J. Haematol. 99, 542-547 which is hereby incorporated as reference).

Test for factor VIII activity:

Suitable assays for testing for factor VIII activity, and thereby providing means for selecting suitable factor VIII variants for use in the present invention, can be performed as simple *in vitro* tests as described, for example, in Kirkwood TBL, Rizza CR, Snape TJ, Rhymes IL, Austen DEG. Identification of sources of interlaboratory variation in factor VIII assay. B J Haematol 1981; 37; 559-68.; or Kessels et al., British Journal of Haematology, Vol. 76 (Suppl.1) pp. 16 (1990)). Factor VIII activity may also be measured by a two-step chromogenic assay based on the amidolytic activity of generated FXa (Wagenvoord et al, 1989, Haemostasis, 19(4):196-204) (*"the chromogenic assay"*).
Factor VIII biological activity may also be quantified by measuring the ability of a preparation to correct the clotting time of factor VIII-deficient plasma, e.g., as described in Nilsson et al., 1959.(Nilsson IM, Blombaeck M, Thilen A, von Francken I., Carriers of haemophilia A - A laboratory study, Acta Med Scan 1959; 165:357). In this assay, biological activity is expressed as units/ml plasma (1 unit corresponds to the amount of FVIII present in normal pooled plasma.

Aspects of the invention:

In one aspect, the invention concerns a pharmaceutical composition comprising a FVII polypeptide and a FVIII polypeptide as the sole active coagulation factors. In one embodiment, the FVII polypeptide is human recombinant FVIIa. In one embodiment, the FVIII polypeptide is human recombinant FVIII. In one embodiment, the FVII polypeptide and the FVIII polypeptide are mixed. In one embodiment, the FVII polypeptide and the FVIII polypeptide are in separate containers. In one embodiment, the composition is for home treatment.
In another aspect, the invention concerns a kit for treatment of bleeding episodes comprising

a) An effective amount of a FVII polypeptide and, optionally, a pharmaceutically acceptable carrier in a first unit dosage form;
b) An effective amount of a FVIII polypeptide and, optionally, a pharmaceutically acceptable carrier in a second unit dosage form; and
c) Container means for containing said first and second dosage forms.

In one embodiment, the FVII polypeptide is human recombinant FVIIa. In one embodiment, the FVIII polypeptide is human recombinant FVIII. In one embodiment, the kit is for home treatment.
In another aspect, the invention concerns the use of a FVII polypeptide and a FVIII polypeptide for the preparation of a medicament for the treatment of bleedings in a subject suffering from a FVIII responsive syndrome. In another aspect, the invention concerns the use of a FVII polypeptide and a FVIII polypeptide for the preparation of a medicament for the treatment of bleedings in a subject having a reduced level of FVIII. In one embodiment, the medicament is for treatment of bleeding episodes in haemophilia A patients. In one embodiment, the medicament comprises a mixture of a FVII polypeptide and a FVIII polypeptide. In one embodiment, the medicament is prepared in the form of a first dosage form comprising a FVII polypeptide and a second dosage form comprising a FVIII polypeptide. In one embodiment, the FVII polypeptide is human recombinant FVIIa. In one embodiment, the FVIII polypeptide is human recombinant FVIII.
In another aspect, the invention concerns a method to enhance haemostasis in a subject suffering from a FVIII responsive syndrome compared to when the subject is treated with FVIII as the only coagulation protein, the method comprising administering to the subject in need thereof an effective amount of a FVII polypeptide and an effective amount of a FVIII polypeptide
In another aspect, the invention concerns a method to enhance haemostasis in a subject having a reduced level of FVIII compared to when the subject is treated with FVIII as the only coagulation protein, the method comprising administering to the subject in need thereof an effective amount of a FVII polypeptide and an effective amount of a FVIII polypeptide.
In another aspect, the invention concerns a method to enhance formation of thrombin in a subject suffering from a FVIII responsive syndrome compared to when the subject is treated with FVIII as the only coagulation protein, the method comprising administering to the subject in need thereof an effective amount of a FVII polypeptide and an effective amount of a FVIII polypeptide.
In another aspect, the invention concerns a method to enhance formation of thrombin in a subject having a reduced level of FVIII compared to when the subject is treated with FVIII as the only coagulation protein, the method comprising administering to the subject in need thereof an effective amount of a FVII polypeptide and an effective amount of a FVIII polypeptide.
In another aspect, the invention concerns a method for reducing the number of administrations of coagulation factor protein needed to accomplish haemostasis in a subject suffering from a FVIII responsive syndrome compared to the number of administrations needed when FVIII is administered to the subject as the only coagulation factor protein, the method comprising administering to a subject in need thereof an effective amount of a FVII polypeptide and an effective amount of a FVIII polypeptide.

In another aspect, the invention concerns a method for reducing the number of administrations of coagulation factor protein needed to accomplish haemostasis in a subject having a reduced level of FVIII compared to the number of administrations needed when FVIII is administered to the subject as the only coagulation factor protein, the method comprising administering to a subject in need thereof an effective amount of a FVII polypeptide and an effective amount of a FVIII polypeptide.

In another aspect, the invention concerns a method for reducing the amount of administered coagulation factor protein needed to accomplish haemostasis in a subject suffering from a FVIII responsive syndrome compared to the amount of administered coagulation factor protein needed when FVIII is administered to the subject as the only coagulation factor protein, the method comprising administering to a subject in need thereof an effective amount of a FVII polypeptide and an effective amount of a FVIII polypeptide.

In another aspect, the invention concerns a method for reducing the amount of administered coagulation factor protein needed to accomplish haemostasis in a subject having a reduced level of FVIII compared to the amount of administered coagulation factor protein needed when FVIII is administered to the subject as the only coagulation factor protein, the method comprising administering to a subject in need thereof an effective amount of a FVII polypeptide and an effective amount of a FVIII polypeptide.

In another aspect, the invention concerns a method of treating bleedings in a subject suffering from a FVIII responsive syndrome, the method comprising administering to the subject in need thereof an effective amount of a FVII polypeptide and a FVIII polypeptide.

In another aspect, the invention concerns a method of treating bleedings in a subject having a reduced level of FVIII, the method comprising administering to the subject in need thereof an effective amount of a FVII polypeptide and a FVIII polypeptide.

In one embodiment of the methods, the FVII polypeptide is human recombinant FVIIa. In one embodiment, the FVIII polypeptide is human recombinant FVIII. In one embodiment, the the subject suffers from haemophilia A.

The present invention is further illustrated by the following examples, which, however, are not to be construed as limiting the scope of protection. The features disclosed in the foregoing description and in the following examples may, both separately and in any combination thereof, be material for realizing the invention in diverse forms thereof.

EXAMPLES

***Example 1: In vivo treatment of a haemophilia patient with intracranial bleeds***

When a non-inhibitor haemophilia A patient suffering from intracranial bleeds is treated with a commercially available FVIII product he will generally need between 10 and 20 injections or infusions of FVIII to achieve haemostasis. The FVIII infusion will intend to achieve an initial FVIII plasma concentration of at least 80 % of normal level followed by a plasma concentration of 50 % for one week.

Such a patient is treated with one dose of 90-180 $\mu$g/kg b.w. of NovoSeven® (Novo Nordisk A/S, Bagsvaerd, Denmark) and a simultaneously administered FVIII product, or with one dose of 90-180 $\mu$g/kg b.w. of NovoSeven® (Novo Nordisk A/S, Bagsvaerd, Denmark) and a FVIII product within a time separation, e.g., 5 minutes. Both products are injected through the same intravenous access. The patient experiences a reduced time to obtain bleeding arrest and a reduced number of injections to maintain haemostasis. This regiment leads to a reduced total amount of coagulation factor protein usage for bleeding arrest and haemostasis.

***Example 2: In vivo treatment of a haemophilia patient with compartment syndrome bleeds***

The patient is a non-inhibitor haemophilia A patient suffering from compartment syndrome bleeds in right upper extremity due to external trauma. When such a patient is treated with a commercially available FVIII product he will generally need between 20 and 40 injections or infusions of FVIII to achieve haemostasis, often in connection with emergency surgery. The FVIII infusion will intend to achieve an initial FVIII plasma concentration of at least 80 to 100 % followed by a plasma concentration of 50 % for 1 to 2 weeks.

Such a patient is treated with one dose of 90-180 $\mu$g/kg b.w. of NovoSeven® (Novo Nordisk A/S, Bagsvaerd, Denmark) and a simultaneously administered FVIII product, or with one dose of 90-180 $\mu$g/kg b.w. of NovoSeven® (Novo Nordisk A/S, Bagsvaerd, Denmark) and a FVIII product within a time separation, e.g., 5 minutes. Both products are injected through the same intravenous access. The patient experiences a reduced time to obtain bleeding arrest and a reduced number of injections to maintain haemostasis. This regiment leads to a reduced total amount of coagulation factor protein usage for bleeding arrest and haemostasis

Just prior to surgery a repeated dose of FVIIa in combination with FVIII may be relevant.

***Example 3: In vivo treatment of a haemophilia patient with upper gastrointestinal bleeds***

The patient is a non-inhibitor haemophilia A patient suffering from gastrointestinal bleeds secondary to NSAID (non steroid anti inflammatory drug) usage. When such a patient is treated with a commercially available FVIII product he will generally need between 20 and 40 injections or infusions of FVIII to achieve haemostasis, often in connection with emergency gastroscopy.

The FVIII infusion will intend to achieve an initial FVIII plasma concentration of at least 80 to 100 % followed by a plasma concentration of 50 % for 5 to 10 days.

Such a patient is treated with one dose of 90-180 $\mu$g/kg b.w. of NovoSeven® (Novo Nordisk A/S, Bagsvaerd, Denmark) and a simultaneously administered FVIII product, or with one dose of 90-180$\mu$ g/kg b.w. of NovoSeven® (Novo Nordisk A/S, Bagsvaerd, Denmark) and a FVIII product within a time separation, e.g., 5 minutes. Both products are injected through the same intravenous access. The patient experiences a reduced time to obtain bleeding arrest and a reduced number of injections to maintain haemostasis. This regiment leads to a reduced total amount of coagulation factor protein usage for bleeding arrest and haemostasis

Just prior to gastroscopy a repeated dose of FVIIa in combination with FVIII may be relevant

***Example 4: In vivo treatment of a multitransfused traumatised patient with diffuse bleeds***

The patient is suffering from diffuse bleeds due to external trauma. Prior to this diffuse bleeding condition the patient has been treated with large amounts of fluids for i.v. injection, colloid infusion products, albumin and red blood cell concentrates. This multitransfused clinical state is characterised by low platelet numbers and low concentration of fibrinogen and FVIII.

Treatment will include transfusion with platelets, fresh frozen plasma and FVIII products. The FVIII infusion will intend to achieve at least 80% of normal level and will be continued until the diffuse bleeding state has resolved.

Such a patient is treated with one dose of 90-180 $\mu$g/kg b.w. of NovoSeven® (Novo Nordisk A/S, Bagsvaerd, Denmark) and a simultaneously administered FVIII product, or with one dose of 90-180 $\mu$g/kg b.w. of NovoSeven® (Novo Nordisk A/S, Bagsvaerd, Denmark) and a FVIII product within a time separation, e.g., 5 minutes. Both products are injected through the same intravenous access. The patient experiences a reduced time to obtain bleeding arrest from multiple bleeding sites and a reduced number of injections to maintain haemostasis. This regiment leads to a reduced total amount of coagulation factor protein usage for bleeding arrest and haemostasis and to improve survival.

Just prior to surgery or invasive procedures a repeated dose of FVIIa in combination with FVIII may be relevant.

***Example 5: Assaying coagulation status of a non-inhibitor haemophilia A patient***

The patient is a non-inhibitor haemophilia A patient suffering from bleeds, e.g., intracranial bleeds.

When such as patient is treated with a commercially available FVIII product he will generally need between 10 and 20 injections or infusions of FVIII to achieve haemostasis. The FVIII infusion will intend to achieve an initial FVIII plasma concentration of at least 80 % of normal level followed by a plasma concentration of 50 % for one week.In vivo assays

Such a patient is treated with one dose of 90-180 $\mu$g/kg b.w. of NovoSeven® (Novo Nordisk A/S, Bagsvaerd, Denmark) and a simultaneously administered FVIII product, or with one dose of 90-180 $\mu$g/kg b.w. of NovoSeven® (Novo Nordisk A/S, Bagsvaerd, Denmark) and a FVIII product within a time separation, e.g., 5 minutes. Both products are injected through the same intravenous access. Ten minutes after administration of the latter of the two coagulation proteins a blood sample are drawn and a whole blood coagulation analysis performed using the thrombo-elastographic method which is a standardised assay, clinical relevant for coagulation status (see, for example, Meh et al., BLOOD COAGULATION & FIBRINOLYSIS 2001;12:627-637). Using standard parameter readings from such an assay enhanced fibrin clot formation, increased clot strenght and prolonged clot lysis time are demonstrated. Such measurement in sequential blood samples demonstrates the variation of these parametres as function of time after injection of the factor VII and the factor VIII products.

***Example 6: Shortening the Clotting Time with Combinations of factors VIIa and factor VIII***

METHODS:

Clot assay: The specific clotting activity of recombinant human coagulation factor VIIa (rFVIIa), in the absence or presence of various concentrations of plasma purified human factor VIII (FVIII) was measured in one-stage assays as previously described (Persson et al., J Biol Chem 276: 29195-9, 2001). In short, aliquots (55 $\mu$l) of rFVIIa (0.2-3 $\mu$g/ml, Novo Nordisk stock) in 50 mM Pipes, 100 mM NaCl, 2 mM EDTA, 1% BSA, pH 7.2, were mixed with an equal volume buffer containing 50 mM CaCl2 and phosphatidylcholine/phosphatidylserine vesicles (total phospholipid concentration 100 μM; 80% phosphatidylcholine/20% phosphatidylserine), and clotting was started by adding 55 μl normal human plasma (Novo Nordisk pooled plasma standard) or FVIII-deficient plasma (Helena Labs Helena Labs #5793) added various concentrations of FVIII ((10, 50, and 80 % of the plasma concentration, Haematologic Technologies). Clotting was followed for 400 seconds in an ACL 300 Research coagulometer (Instrumentation Laboratory, Milan, Italy) using the standard APTT program.

RESULTS:

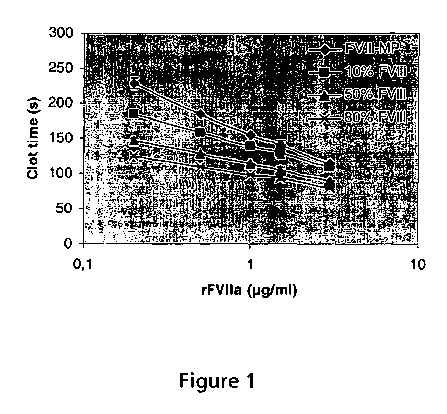

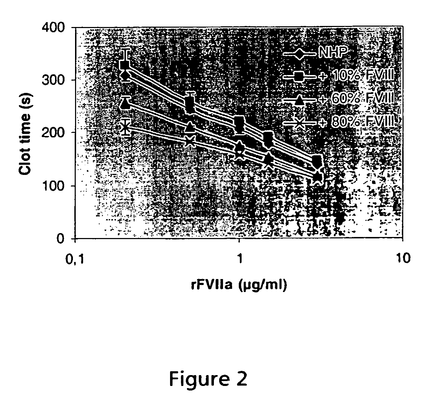

Clot assay: rFVIIa and FVIII, separately and in combination was added to FVIII-deficient and normal human plasma and the clotting times was determined. Prior to addition of rFVIIa/FVIII the clotting time of both plasmas was longer than the 400 seconds monitoring time. The clot shortening effect of rFVIIa in the absence and presence of FVIII in FVIII-deficient and normal human plasma is shown in figure 1 and 2, respectively.

CONCLUSION:

These results demonstrate that the combination of rFVIIa and FVIII is capable of shortening the clotting time of both FVIII-deficient and normal plasma beyond what is seen when the proteins was added separately.

**Claims**

1. A pharmaceutical composition comprising:

    (a) a preparation of human factor VIIa or an amino acid variant of human factor VIIa having no more than 20 amino acids replaced, deleted or inserted compared to wild-type factor VIIa, and
    (b) a preparation of human factor VIII or a truncated form of human factor VIII, wherein (a) and (b) are the sole haemostatic agents of the composition.

2. A composition according to claim 1, wherein the ratio between the activity of the factor VIIa variant and the activity of native human factor VIIa (wild-type FVIIa) is at least about 1.25 when tested in the "In Vitro Hydrolysis Assay" as described herein.

3. A composition according to claim 1, wherein the human factor VIIa is recombinant human factor VIIa.

4. A composition according to any one of claims 1-3, wherein the human factor VIII is recombinant human factor VIII.

5. A composition according to claim 1, wherein the truncated form of human FVIII is a B-domain deleted human factor VIII.

6. A composition according to any one of claims 1-5, wherein the human factor VIIa polypeptide and the human factor VIII polypeptide are present in a ratio by mass of between about 100:1 1 and about 1:100 (w/w factor VII : factor VIII).

7. A composition according to any one of claims 1-6, wherein the composition is for treatment of bleeding episodes in a subject.

8. A kit of parts for treatment of bleeding episodes in a subject, comprising:

    a) an effective amount of a preparation of human factor VIIa or an amino acid variant of human factor VIIa having no more than 20 amino acids replaced, deleted or inserted compared to wild-type factor VIIa, and a pharmaceutically acceptable carrier in a first unit dosage form;
    b) an effective amount of a preparation of human factor VIII or a truncated form of human factor VIII, and a pharmaceutically acceptable carrier in a second unit dosage form; and
    c) container means for containing said first and second dosage forms,

    wherein (a) and (b) are the sole haemostatic agents of the kit.

9. A kit according to claim 8, wherein the ratio between the activity of the human factor VIIa variant and the activity of native human factor VIIa (wild-type FVIIa) is at least about 1.25 when tested in the "In Vitro Hydrolysis Assay" as described in the present description.

**10.** A kit according to claim 8, wherein the human factor human VIIa is recombinant human factor VIIa.

**11.** A kit according to any of claims 8-10, wherein the human factor VIII is recombinant human factor VIII.

**12.** A kit according to claim 8, wherein the truncated human FVIII is a B-domain deleted human factor VIII.

**13.** A kit according to any one of claims 8-12, wherein the factor VIIa polypeptide and the human factor VIII polypeptide are present in a ratio by mass of between about 100.1 and about 1:100 (w/w factor VIIa:factor VIII).

**14.** A kit according to any one of claims 8-14, wherein the composition is for treatment of bleeding episodes in a subject with a reduced level of factor VIII, preferably for the treatment of bleedings episodes in subjects having haemophilia A.

**15.** Use of a preparation of (a) human factor VIIa or an amino acid variant of human factor VIIa having no more than 20 amino acids replaced, deleted or inserted compared to wild-type factor VIIa in combination with a preparation of (b) human factor VIII or a truncated form of human factor VIII as the sole haemostatic agents for the manufacture of a medicament for treating bleeding episodes in a subject.

**16.** Use according to claim 15, wherein the ratio between the activity of the human factor VIIa variant and the activity of native human factor VIIa (wild-type FVIIa) is at least about 1.25 when tested in the "In Vitro Hydrolysis Assay" as described in the present description.

**17.** Use according to claim 15, wherein the human factor VIIa is recombinant human factor VIIa.

**18.** Use according to claim 15, wherein the human factor VIII is recombinant human factor VIII.

**19.** Use according to claim 16, wherein the truncated human FVIII is a B-domain deleted human factor VIII.

**20.** Use according to any one of claims 15-19, wherein the medicament is in a single-dosage form.

**21.** Use according to any one of claims 15-19, wherein the medicament is prepared in the form of a first dosage form comprising a preparation human factor VIIa or an amino acid variant of human factor VIIa having no more than 20 amino acids replaced, deleted or inserted compared to wild-type factor VIIa, and a second dosage form comprising a preparation of human factor VIII or a truncated form of human factor VIII.

**Patentansprüche**

**1.** Arzneimittel, umfassend

(a) ein Präparat von Humanfaktor VIIa oder einer Aminosäurevariante von Humanfaktor VIIa mit, verglichen mit dem Faktor VIIa vom Wildtyp, nicht mehr als 20 ersetzten, deletierten oder eingefügten Aminosäuren und
(b) ein Präparat von Humanfaktor VIII oder einer verkürzten Form von Humanfaktor VIII,

wobei (a) und (b) die einzigen Hämostatika des Mittels sind.

**2.** Mittel nach Anspruch 1, wobei das Verhältnis zwischen der Aktivität der Faktor-VIIa-Variante und der Aktivität von nativem Humanfaktor VIIa (FVIIa vom Wildtyp) beim Testen im wie hier beschriebenen "In-vitro-Hydrolyseassay" mindestens etwa 1,25 beträgt.

**3.** Mittel nach Anspruch 1, wobei der Humanfaktor VIIa rekombinanter Humanfaktor VIIa ist.

**4.** Mittel nach einem der Ansprüche 1-3, wobei der Humanfaktor VIII rekombinanter Humanfaktor VIII ist.

**5.** Mittel nach Anspruch 1, wobei die verkürzte Form von Human-FVIII ein B-Domänen-deletierter Humanfaktor VIII ist.

**6.** Mittel nach einem der Ansprüche 1-5, wobei das Humanfaktor-VIIa-Polypeptid und das Humanfaktor-VIII-Polypeptid in einem Massenverhältnis zwischen etwa 100:1 und etwa 1:100 (G/G Faktor VII:Faktor VIII) vorliegen.

**7.** Mittel nach einem der Ansprüche 1-6, wobei das Mittel der Behandlung von Blutungsepisoden bei einem Probanden dient.

**8.** Kit aus Teilen zur Behandlung von Blutungsepisoden bei einem Probanden, umfassend:

(a) eine wirksame Menge eines Präparats von Humanfaktor VIIa oder einer Aminosäurevariante von Humanfaktor VIIa mit, verglichen mit dem Faktor VIIa vom Wildtyp, nicht mehr als 20 ersetzten, deletierten oder eingefügten Aminosäuren und einen pharmazeutisch verträglichen Träger in einer ersten Dosierungseinheitsform;
(b) eine wirksame Menge eines Präparats von Humanfaktor VIII oder einer verkürzten Form von Humanfaktor VIII und einen pharmazeutisch verträglichen Träger in einer zweiten Dosierungseinheitsform; und
(c) Behältermittel zum Aufnehmen der ersten und der zweiten Dosierungsform,

wobei (a) und (b) die einzigen Hämostatika des Kits sind

**9.** Kit nach Anspruch 8, wobei das Verhältnis zwischen der Aktivität der Humanfaktor-VIIa-Variante und der Aktivität von nativem Humanfaktor VIIa (FVIIa vom Wildtyp) beim Testen im wie in der vorliegenden Beschreibung beschriebenen "In-vitro-Hydrolyseassay" mindestens etwa 1,25 beträgt.

**10.** Kit nach Anspruch 8, wobei der Humanfaktor VIIa rekombinanter Humanfaktor VIIa ist.

**11.** Kit nach einem der Ansprüche 8-10, wobei der Humanfaktor VIII rekombinanter Humanfaktor VIII ist.

**12.** Kit nach Anspruch 8, wobei der verkürzte Human-FVIII ein B-Domänen-deletierter Humanfaktor VIII ist.

**13.** Kit nach einem der Ansprüche 8-12, wobei das Faktor-VIIa-Polypeptid und das Humanfaktor-VIII-Polypeptid in einem Massenverhältnis zwischen etwa 100:1 und etwa 1:100 (G/G Faktor VII:Faktor VIII) vorliegen.

**14.** Kit nach einem der Ansprüche 8-14, wobei das Mittel der Behandlung von Blutungsepisoden bei einem Probanden mit einem reduzierten Faktor-VIII-Spiegel, vorzugsweise der Behandlung von Blutungsepisoden in Probanden mit Hämophilie A dient.

**15.** Verwendung eines Präparats von (a) Humanfaktor VIIa oder einer Aminosäurevariante von Humanfaktor VIIa mit, verglichen mit dem Faktor VIIa vom Wildtyp, nicht mehr als 20 ersetzten, deletierten oder eingefügten Aminosäuren in Kombination mit einem Präparat von Humanfaktor VIII oder einer verkürzten Form von Humanfaktor VIII als die einzigen Hämostatika des Mittels zur Herstellung eines Medikaments zu Behandlung von Blutungsepisoden bei einem Probanden.

**16.** Verwendung nach Anspruch 15, wobei das Verhältnis zwischen der Aktivität der Humanfaktor-VIIa-Variante und der Aktivität von nativem Humanfaktor VIIa (FVIIa vom Wildtyp) beim Testen im wie in der vorliegenden Beschreibung beschriebenen "In-vitro-Hydrolyseassay" mindestens etwa 1,25 beträgt.

**17.** Verwendung nach Anspruch 15, wobei der Humanfaktor VIIa rekombinanter Humanfaktor VIIa ist.

**18.** Verwendung nach Anspruch 15, wobei der Humanfaktor VIII rekombinanter Humanfaktor VIII ist.

**19.** Verwendung nach Anspruch 16, wobei der verkürzte Human-FVIII ein B-Domänen-deletierter Humanfaktor VIII ist.

**20.** Verwendung nach einem der Ansprüche 15-19, wobei das Medikament in einer Einzeldosierungsform vorliegt.

**21.** Verwendung nach einem der Ansprüche 15-19, wobei das Medikament in Form einer ersten Dosierungsform, umfassend ein Präparat von Humanfaktor VIIa oder einer Aminosäurevariante von Humanfaktor VIIa mit, verglichen mit dem Faktor VIIa vom Wildtyp, nicht mehr als 20 ersetzten, deletierten oder eingefügten Aminosäuren, und eine zweite Dosierungsform, umfassend ein Präparat von Humanfaktor VIII oder einer verkürzten Form von Humanfaktor VIII, hergestellt wird.

**Revendications**

1. Composition pharmaceutique comprenant :

   (a) une préparation du facteur VIIa humain ou d'un variant, portant sur les acides aminés, du facteur VIIa humain, n'ayant pas plus de 20 acides aminés remplacés, délétés ou insérés par comparaison avec le facteur VIIa de type sauvage, et
   (b) une préparation du facteur VIII humain ou d'une forme tronquée du facteur VIII humain,

   dans laquelle (a) et (b) sont les seuls agents hémostatiques de la composition.

2. Composition selon la revendication 1, dans laquelle le rapport entre l'activité du variant du facteur VIIa et l'activité du facteur VIIa humain natif (FVIIa de type sauvage) est d'au moins environ 1,25, quand il est déterminé dans l'" analyse par hydrolyse in vitro", telle que décrite dans l'invention.

3. Composition selon la revendication 1, dans laquelle le facteur VIIa humain est un facteur VIIa humain recombinant.

4. Composition selon l'une quelconque des revendications 1 à 3, dans laquelle le facteur VIII humain est un facteur VIII humain recombinant.

5. Composition selon la revendication 1, dans laquelle la forme tronquée du FVIII humain est un facteur VIII humain délété du domaine B.

6. Composition selon l'une quelconque des revendications 1 à 5, dans laquelle le polypeptide du facteur VIIa humain et le polypeptide du facteur VIII humain sont présents selon un rapport pondéral compris entre environ 100:1 et environ 1:100 (p/p facteur VII : facteur VIII).

7. Composition selon l'une quelconque des revendications 1 à 6, cette composition étant destinée au traitement des crises hémorragiques chez un sujet.

8. Trousse de pièces pour le traitement des crises hémorragiques chez un sujet, comprenant :

   a) une quantité efficace d'une préparation du facteur VIIa humain ou d'un variant du facteur VIIa humain portant sur les acides aminés, n'ayant pas plus de 20 acides aminés remplacés, délétés ou insérés par comparaison avec le facteur VIIa de type sauvage, et un support acceptable d'un point de vue pharmaceutique, dans une première forme posologique unitaire ;
   b) une quantité efficace d'une préparation du facteur VIII humain ou d'une forme tronquée du facteur VIII humain, et un support acceptable d'un point de vue pharmaceutique, dans une deuxième forme posologique unitaire ; et
   c) un moyen contenant, destiné à contenir lesdites première et deuxième formes posologiques,

   dans laquelle (a) et (b) sont les seuls agents hémostatiques de la trousse.

9. Trousse selon la revendication 8, dans laquelle le rapport entre l'activité du variant du facteur VIIa humain et l'activité du facteur VIIa humain natif (FVIIa de type sauvage) est d'au moins 1,25, quand il est déterminé dans l'analyse par hydrolyse in vitro" telle que décrite dans la présente invention.

10. Trousse selon la revendication 8, dans laquelle le facteur VIIa humain est un facteur VIIa humain recombinant.

11. Trousse selon l'une quelconque des revendications 8 à 10, dans laquelle le facteur VIII l humain est un facteur VIII humain recombinant.

12. Trousse selon la revendication 8, dans laquelle le FVIII humain tronqué est un facteur VIII humain délété du domaine B.

13. Trousse selon l'une quelconque des revendications 8 à 12, dans laquelle le polypeptide du facteur VIIa et le polypeptide du facteur VIII humain sont présents selon un rapport pondéral compris entre environ 100:1 et environ 1:100 (p/p facteur VIIa : facteur VIII).

14. Trousse selon l'une quelconque des revendications 8 à 13, dans laquelle la composition est destinée au traitement des crises hémorragiques chez un sujet présentant un taux réduit du facteur VIII, de préférence au traitement des crises hémorragiques chez des sujets présentant une hémophilie A.

15. Utilisation d'une préparation (a) du facteur VIIa humain ou d'un variant portant sur les acides aminés du facteur VIIa humain, n'ayant pas plus de 20 acides aminés remplacés, délétés ou insérés par comparaison avec le facteur VIIa de type sauvage, en combinaison avec une préparation (b) du facteur VIII humain ou d'une forme tronquée du facteur VIII humain en tant qu'agents hémostatiques uniques, pour la fabrication d'un médicament destiné au traitement des crises hémorragiques chez un sujet.

16. Utilisation selon la revendication 15, pour laquelle le rapport entre l'activité du variant du facteur VIIa humain et l'activité du facteur VIIa humain natif (FVIIa de type sauvage) est d'au moins 1,25 quand il est déterminé dans l'" analyse par hydrolyse in vitro", telle que décrite dans la présente invention.

17. Utilisation selon la revendication 15, pour laquelle le facteur VIIa humain est un facteur VIIa humain recombinant.

18. Utilisation selon la revendication 15, pour laquelle le facteur VIII humain est un facteur VIII humain recombinant.

19. Utilisation selon la revendication 16, pour laquelle le FVIII humain tronqué est un facteur VIII humain délété du domaine B.

20. Utilisation selon l'une quelconque des revendications 15 à 19, pour laquelle le médicament se présente sous une forme posologique unitaire.

21. Utilisation selon l'une quelconque des revendications 15 à 19, pour laquelle le médicament est préparé sous forme d'une première forme posologique comprenant une préparation du facteur VIIa humain ou d'un variant portant sur les acides aminés du facteur VIIa humain, n'ayant pas plus de 20 acides aminés remplacés, délétés ou insérés par comparaison avec le facteur VIIa de type sauvage, et d'une deuxième forme posologique comprenant une préparation du facteur VIII humain ou d'une forme tronquée du facteur VIII humain.

**Figure 1**

Figure 2

## REFERENCES CITED IN THE DESCRIPTION

**Patent documents cited in the description**

- EP 225160 A
- EP 82182 A
- US 5891843 A
- US 4784950 A
- US 5580560 A
- US 5997864 A

- EP 200421 A
- US 4456591 A
- US 4757006 A
- US 4965199 A
- US 4837028 A
- US 4501728 A
- US 4975282 A

**Non-patent literature cited in the description**

- **LUSHER et al.** *Haemophilia,* 1998, vol. 4, 790-798
- **LINO et al.** *Arch. Biochem. Biophys.,* 1998, vol. 352, 182-192
- **MOLLERUP et al.** *Biotechnol. Bioeng.,* 1995, vol. 48, 501-505
- **KORNFELT et al.** *Arch. Biochem. Biophys.,* 1999, vol. 363, 43-54
- **WILDGOOSE et al.** *Biochem,* 1990, vol. 29, 3413-3420
- **KAZAMA et al.** *J. Biol. Chem.,* 1995, vol. 270, 66-72
- **HOLST et al.** *Eur. J. Vasc. Endovasc. Surg.,* 1998, vol. 15, 515-520
- **NICOLAISEN et al.** *FEBS Letts.,* 1993, vol. 317, 245-249
- **PERSSON et al.** *J. Biol. Chem.,* 1997, vol. 272, 19919-19924
- **PERSSON.** *FEBS Letts.,* 1997, vol. 413, 359-363
- **TOOLE et al.** *Nature,* 1984, vol. 312, 342-347
- **FULCHER et al.** *Proc. Acad. Nat. Sci. USA,* 1982, vol. 79, 1648-1652
- **ROTBLAT et al.** *Biochemistry,* 1985, vol. 24, 4294-4300
- **FASS et al.** *Blood,* 1982, vol. 59, 594-600
- **KNUTSON et al.** *Blood,* 1982, vol. 59, 615-624
- **LOLLAR et al.** *Blood,* 2000, vol. 95 (2), 564-568
- **LOLLAR et al.** *Blood,* 2001, vol. 97 (1), 169-174
- **MEH et al.** *Blood Coagulation & Fibrinolysis,* 2001, vol. 12, 627-637
- **VIG et al.** *Hematology,* 2001, vol. 6 (3), 205-213

- **VIG et al.** *Blood coagulation & fibrinolysis,* October 2001, vol. 12 (7), 555-561
- **GLIDDEN et al.** *Clinical and applied thrombosis/hemostasis,* October 2000, vol. 6 (4), 226-233
- **MCKENZIE et al.** *Cardiology,* April 1999, vol. 92 (4), 240-247
- **DAVIS et al.** *Journal of the American Society of Nephrology,* 1995, vol. 6 (4), 1250-1255
- **CARR ME, ZEKERT SL.** Measurement of platelet-mediated force development during plasma clot formation. *AM J MED SCI,* 1991, vol. 302, 13-8
- *Am J Clin Pathol,* 1961, vol. 36, 212
- **HAGEN et al.** *Proc.Natl.Acad.Sci. USA,* 1986, vol. 83, 2412-2416
- **BROZE ; MAJERUS.** *J.Biol.Chem.,* 1980, vol. 255 (4), 1242-1247
- **HEDNER ; KISIEL.** *J.Clin.Invest.,* 1983, vol. 71, 1836-1841
- **BJOERN et al.** *Research Disclosure,* September 1986, vol. 269, 564-565
- **CUNNINGHAM ; WELLS.** *Science,* 1989, vol. 244, 1081-1085
- **DE VOS et al.** *Science,* 1992, vol. 255, 306-312
- **SMITH et al.** *Journal of Molecular Biology,* 1992, vol. 224, 899-904
- **WIODAVER et al.** *FEBS Letters,* 1992, vol. 309, 59-64
- **WAKABAYASHI et al.** *J. Biol. Chem.,* 1986, vol. 261, 11097
- **THIM et al.** *Biochem.,* 1988, vol. 27, 7785
- **OSTERUD et al.** *Biochem.,* 1972, vol. 11, 2853
- **HEDNER et al.** *J. Clin. Invest.,* 1983, vol. 71, 1836
- Remington's Pharmaceutical Sciences. Mack Publishing Co, 1990

- **MONROE et al.** *Brit. J. Haematol.,* 1997, vol. 99, 542-547
- **B J HAEMATOL.** *B J Haematol,* 1981, vol. 37, 559-68
- **KESSELS et al.** *British Journal of Haematology,* 1990, vol. 76, 16
- **WAGENVOORD et al.** *Haemostasis,* 1989, vol. 19 (4), 196-204
- **MEH et al.** *BLOOD COAGULATION & FIBRINOLYSIS,* 2001, vol. 12, 627-637
- **PERSSON et al.** *J Biol Chem,* 2001, vol. 276, 29195-9